# EUROPÄISCHE PATENTSCHRIFT

(11) **EP 0 970 044 B1**
(45) Veröffentlichungstag und Bekanntmachung des Hinweises auf die Patenterteilung: **07.05.2003**
(21) Anmeldenummer: 98913580.1
(22) Anmeldetag: 23.02.1998
(51) Int. Cl.: C07C 255/31, C07C 255/60, C07C 233/08, C07C 233/13, C07C 233/19, A01N 37/34

(54) **CYCLOALKYLALKANCARBONSÄUREAMIDE, DEREN HERSTELLUNG UND VERWENDUNG**
CYCLOALKYLALKANECARBOXAMIDES AND THE PRODUCTION AND USE THEREOF
AMIDES D'ACIDE CYCLOALKYLALCANECARBOXYLIQUE, LEUR PRODUCTION ET LEUR UTILISATION

(30) Priorität: 14.03.1997 DE 19710618
(43) Veröffentlichungstag der Anmeldung: 12.01.2000
(73) Patentinhaber: BASF AKTIENGESELLSCHAFT, 67056 Ludwigshafen (DE)
(72) Erfinder: WETTERICH, Frank, D-67112 Mutterstadt (DE); EICKEN, Karl, D-67157 Wachenheim (DE); LORENZ, Gisela, D-67434 Neustadt (DE); AMMERMANN, Eberhard, D-64646 Heppenheim (DE); STRATHMANN, Siegfried, D-67117 Limburgerhof (DE)
(86) Internationale Anmeldenummer: EP9801031
(87) Internationale Veröffentlichungsnummer: WO98041499

(56) Entgegenhaltungen:
- EP-A- 0 629 627
- DE-A- 4 320 223
- US-A- 2 538 322
- US-A- 4 946 867
- DATABASE CHEMABS CHEMICAL ABSTRACTS SERVICE, COLUMBUS, OHIO, US STN, XP002072796 & JP 58 029 751 A (SUMITOMO CHEMICAL CO LTD) in der Anmeldung erwähnt
- R.W.J. CARNEY ET AL: ORG. PREP. PROCED. INT., Bd. 5, Nr. 1, 1973, Seiten 25-29, XP002072790 in der Anmeldung erwähnt
- L. WESSJOHANN ET AL: CHEM. BER., Bd. 125, Nr. 4, 1992, Seiten 867-882, XP002072931
- DATABASE CHEMABS CHEMICAL ABSTRACTS SERVICE, COLUMBUS, OHIO, US STN, accession no. 95:115258, XP002073738 -& CHEMICAL ABSTRACTS, vol. 95, no. 13, 28.September 1981 Columbus, Ohio, US; abstract no. 115258, XP002073737 & JP 56 039 064 A (MEIJI SEIKA KAISHA LTD) 14.April 1981
- P. RENAUD ET AL: J. ORG. CHEM., Bd. 53, Nr. 16, 1988, Seiten 3745-3752, XP002073736
- B. GIESE ET AL: CHEM. BER., Bd. 119, Nr. 4, 1986, Seiten 1291-1300, XP002072932
- M. SEMONSKY ET AL: COLLECTION CZECHOSLOV. CHEM. COMMUN., Bd. 25, 1960, Seiten 2038-2044, XP002072794
- M. SCHWARZ ET AL: J. ECON. ENTOMOL., Bd. 63, Nr. 2, 1970, Seiten 429-432, XP002072795
- L.H. BRIGGS ET AL: J. CHEM. SOC., C, Nr. 14, 1970, Seiten 1885-1889, XP002072793
- B. GIESE ET AL: CHEM. BER., Bd. 114, Nr. 6, 1981, Seiten 2138-2145, XP002072933
- N.A. PORTER ET AL: TETRAHDRON, Bd. 52, Nr. 12, 1996, Seiten 4181-4198, XP002072791
- F. FAUSTINI ET AL: TETRAHEDRON LETT., Bd. 22, Nr. 45, 1981, Seiten 4533-4536, XP002072792
- F. BUZZETTI ET AL: TETRAHEDRON LETT., Bd. 24, Nr. 24, 1983, Seiten 2505-2508, XP002072934
- W.T. BRADY ET AL: J. AM. CHEM. SOC., Bd. 94, Nr. 12, 1972, Seiten 4278-4284, XP002072935

## Beschreibung

Die vorliegende Erfindung betrifft neue Cycloalkylalkancarbonsäureamide der allgemeinen Formel I 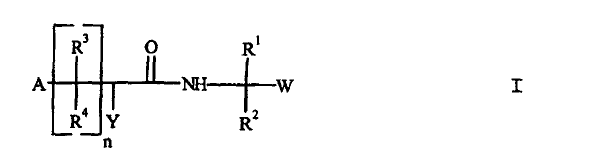 in der die Substituenten die folgende Bedeutung haben:
- A: C₃-C₆-Cycloalkyl, welches einen bis drei Substituenten ausgewählt aus der Gruppe bestehend aus Halogen und C₁-C₃-Alkyl tragen kann;
- R¹: C₁-C₆-Alkyl oder C₂-C₆-Alkenyl, wobei diese Reste partiell oder vollständig halogeniert sein und/oder eine oder zwei der folgenden Gruppen tragen können: C₁-C₄-Alkoxy, C₁-C₄-Halogenalkoxy, C₁-C₄-Alkylthio, C₁-C₄-Alkoxycarbonyl, C₃-C₆-Cycloalkyl und Phenyl, wobei das Phenyl partiell oder vollständig halogeniert sein und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy, C₁-C₄-Alkylthio, C₃-C₆-Cycloalkyl oder Heterocyclyl;
- R², R³, R⁴: Wasserstoff oder unabhängig von jener eine der Bedeutungen des Restes R¹;
- n: 1;
- Y: Cyano oder Halogen;
- W: Phenyl, welches eine bis drei der folgenden Gruppen tragen kann: Nitro, Halogen, Cyano, C₁-C₄-Alkyl, C₁-C₄-Halogenalkyl, C₁-C₄-Alkoxy, C₁-C₄-Halogenalkoxy, C₁-C₄-Alkylthio, C₃-C₆-Cycloalkyl und C₁-C₄-Alkoxycarbonyl.

Weiterhin betrifft die vorliegende Erfindung

Cycloalkylalkancarbonsäureamide der allgemeinen Formel I 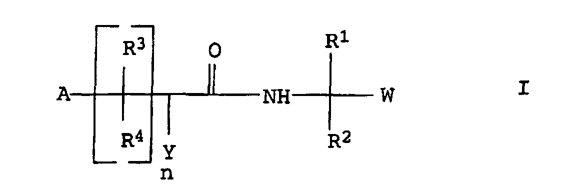 in der die Substituenten die folgende Bedeutung haben:
- A: C₃-C₆-Cycloalkyl, welches einen bis drei Substituenten ausgewählt aus der Gruppe bestehend aus Halogen und C₁-C₃-Alkyl tragen kann;
- R¹: C₁-C₆-Alkyl oder C₂-C₆-Alkenyl, wobei diese Reste partiell oder vollständig halogeniert sein und/oder eine oder zwei der folgenden Gruppen tragen können: C₁-C₄-Alkoxy, C₁-C₄-Halogenalkoxy, C₁-C₄-Alkylthio, C₁-C₄-Alkoxycarbonyl, C₃-C₆-Cycloalkyl und Phenyl, wobei das Phenyl partiell oder vollständig halogeniert sein und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy, C₁-C₄-Alkylthio, C₃-C₆-Cycloalkyl oder Heterocyclyl;
- R²,R³,R⁴: Wasserstoff oder unabhängig von jener eine der Bedeutungen des Restes R¹;
- n: 0 oder 1;
- Y: Cyano oder Halogen;
- W: Naphthyl oder Heteroaryl, wobei diese Reste eine bis drei der folgenden Gruppen tragen können: Nitro, Halogen, Cyano, C₁-C₄-Alkyl, C₁-C₄-Halogenalkyl, C₁-C₄-Alkoxy, C₁-C₄-Halogenalkoxy, C₁-C₄-Alkylthio, C₃-C₆-Cycloalkyl und C₁-C₄-Alkoxycarbonyl,
ausgenommen von Verbindungen der Formel I , wobei
- n: 0 und
- A: Cyclopentyl ist.

Aus der Literatur sind bereits α-Halogen- und α-Cyan-substituierte Carbonsäureamide zur Bekämpfung von Schadpilzen, insbesondere zur Bekämpfung von *Pyricularia oryzae* bekannt (JP-A 57 185202, JP-A 57 188552, JP-A 57 188551, JP-A 58 029751, JP-A 58 029752, WO 95/31432, JP-A 07 206608, JP-A 07 330511, JP-A 08 012508 und US 4,946,867). In J. Pestic. Sci. 12, 79 - 84 (1987) werden Arbeiten, die die bis dato erschienenen α-Halogen-substi-tuierten Carbonsäureamide betreffen, zusammengefaßt. Ferner wird dort der Versuch unternommen, quantitative Struktur-Wirkungs-Beziehungen für diese Fungizid-Klasse aufzustellen.

In der US 4,946,867 wird ein Cyanacetamid-Derivat mit α-ständiger Cyclopentylgruppe, N-[1-(4-Chlorophenyl)ethyl]-2-cyano-2-cyclopentylethanamid, erwähnt.

Da die fungiziden Eigenschaften der bekannten Verbindungen, hinsichtlich ihrer Aktivität gegen Schadpilze wie z.B. *Pyricularia oryzae,* nicht immer voll befriedigen, war es die Aufgabe der vorliegenden Erfindung neue, gegen Schadpilze wie z.B. *Pyricularia oryzae* wirksamere Carbonsäureamide zu finden.

Demgemäß wurden die eingangs definierten neuen, Cycloalkylalkancarbonsäureamide I gefunden. Desweiteren wurden Verfahren zur Herstellung der Verbindungen I und die zu deren Herstellung benötigten Zwischenprodukte der Formel II gefunden. Es wurden Mittel gefunden, die die Verbindungen I enthalten, Verfahren zur Bekämpfung von Schadpilzen mit den Verbindungen I und schließlich die Verwendung der Verbindungen I zur Bekämpfung von Schadpilzen.

Die Verbindungen der Formel I können je nach Substitutionsmuster ein oder mehrere Chiralitätszentren enthalten. Sie liegen dann als Enantiomeren- und Diastereomerengemische vor. Gegenstand der Erfindung sind sowohl die reinen Enantiomeren oder Diastereomeren als auch deren Gemische.

Bei der eingangs angegebenen Definition der Verbindungen I wurden für die Reste R¹ bis R⁴, A und Z Sammelbegriffe verwendet, die für individuelle Aufzählungen der einzelnen Gruppenmitglieder stehen. Die Reste Alkyl, Alkylthio, Alkoxy, Alkoxycarbonyl und Alkenyl können geradkettig oder verzweigt sein.

Der Rest Cycloalkyl bedeutet, sofern keine spezifische Substitution eines Wasserstoffatoms durch Halogen oder C₁-C₃-Alkyl angegeben ist, den unsubstituierten Grundkörper. Die Restedefinition Cyclopentyl beispielsweise entspricht der Summenformel C₅H₁₀.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome ersetzt sein können. Die Bedeutung Halogen steht jeweils für Fluor, Chlor, Brom oder Jod.

Ferner bedeuten beispielsweise:
- C₁-C₄-Alkyl, sowie die Alkylteile von C₁-C₄-Alkylthio:
   Methyl, Ethyl, Propyl, 1-Methylethyl, Butyl, 1-Methylpropyl, 2-Methylpropyl und 1,1-Dimethylethyl;
- C₁-C₆-Alkyl:
   C₁-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Halogenalkyl:
   einen C₁-C₄-Alkylrest wie vorstehend genannt, der partiell oder vollständig durch Fluor, Chlor, Brom und/oder Jod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Jodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₄-Alkoxy sowie die Alkoxyteile von C₁-C₄-Alkoxycarbonyl:
   Methoxy, Ethoxy, Propoxy, 1-Methylethoxy, Butoxy, 1-Methylpropoxy, 2-methylpropoxy und 1,1-Dimethylethoxy;
- C₁-C₄-Halogenalkoxy: einen C₁-C₄-Alkoxyrest wie voranstehend genannt, der partiell oder vollständig durch Fluor, Chlor, Brom und/oder Jod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-Jodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₂-C₆-Alkenyl: Ethylen,Prop-1-en-1-yl, Prop-2-en-1-yl, 1-Methylethenyl Buten-1-yl, Buten-2-yl, Buten-3-yl, 1-Methyl-prop-1-en-1-yl, 2-Methyl-prop-1-en-1-yl, 1-Methylprop-2-en-1-yl und 2-Methyl-prop-2-en-1-yl, Penten-1-yl, Penten-2-yl, Penten-3-yl, Penten-4-yl, 1-Methylbut-1-en-1-yl, 2-Methyl-but-1-en-1-yl, 3-Methylbut-1-en-1-yl, 1-Methyl-but-2-en-1-yl, 2-Methylbut-2-en-1-yl, 3-Methyl-but-2-en-1-yl, 1-Methylbut-3-en-1-yl, 2-Methyl-but-3-en-1-yl, 3-Methylbut-3-en-1-yl, 1,1-Dimethyl-prop-2-en-1-yl, 1,2-Dimethylprop-1-en-1-yl, 1,2-Dimethyl-prop-2-en-1-yl, 1-Ethylprop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 2,1-Dimethyl-but-2-en-1-yl, 1,1-Dimethyl-but-3-en-1-yl, 1,2-Dimethyl-but-1-en-1-yl, 1,2-Dimetyl-but-2-en-1-yl, 1,2-Dimethyl-but-3-en-1-yl, 1,3-Dimetyl-but-1-en-1-yl, 1,3-Dimethyl-but-2-en-1-yl, 1,3-Dimetyl-but-3-en-1-yl, 2,2-Dimethyl-but-3-en-1-yl, 2,3-Dimetyl-but-1-en-1-yl, 2,3-Dimethyl-but-2-en-1-yl, 2,3-Dimetyl-but-3-en-1-yl, 3,3-Dimethyl-but-1-en-1-yl, 3,3-Dimetyl-but-2-en-1-yl, 1-Ethyl-but-1-en-1-yl, 1-Ethyl-but-2-en-1-yl, 1-Ethylbut-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methylprop-1-en-1-yl und 1-Ethyl-2-methyl-prop-2-en-1-yl;
- C₃-C₆-Cycloalkyl: Cyclopropyl, Cyclobutyl, Cyclopentyl, Cyclohexyl;

Heteroaryl: aromatische mono- oder polycyclische Reste, welche neben Kohlenstoffringgliedern zusätzlich 1 bis 4 Stickstoffatome oder 1 bis 3 Stickstoffatome und ein Sauerstoff- oder ein Schwefelatom oder ein Sauerstoff- oder ein Schwefelatom enthalten können, z.B.:
- 5-gliedriges Heteroaryl, enthaltend 1 bis 3 Stickstoffatome: 5-Ring-Heteroarylgruppen, welche neben Kohlenstoffatomen 1 bis 3 Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;
- 5-gliedriges Heteroaryl, enthaltend 1 bis 4 Stickstoffatome oder 1 bis 3 Stickstoffatome und 1 Schwefelatom oder Sauerstoffatom oder 1 Sauerstoff- oder 1 Schwefelatom: 5-Ring-Heteroarylgruppen, welche neben Kohlenstoffatomen 1 bis 4 Stickstoffatome oder 1 bis 3 Stickstoffatome und 1 Schwefeloder Sauerstoffatom oder 1 Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl;
- benzokondensiertes 5-gliedriges Heteroaryl, enthaltend 1 bis 3 Stickstoffatome oder 1 Stickstoffatom und/oder ein Sauerstoff- oder Schwefelatom: 5-Ring-Heteroarylgruppen, welche neben Kohlenstoffatomen 1 bis 4 Stickstoffatome oder 1 bis 3 Stickstoffatome und 1 Schwefel- oder Sauerstoffatom oder 1 Sauerstoff- oder ein Schwefelatom als Ringglieder enthalten können, und in welchen 2 benachbarte Kohlenstoffringglieder oder 1 Stickstoff- und 1 benachbartes Kohlenstoffringglied durch eine Buta-1,3-dien-1,4-diylgruppe verbrückt sein können;
- über Stickstoff gebundenes 5-gliedriges Heteroaryl, enthaltend 1 bis 4 Stickstoffatome, oder über Stickstoff gebundenes benzokondensiertes 5-gliedriges Heteroaryl, enthaltend 1 bis 3 Stickstoffatome: 5-Ring-Heteroarylgruppen, welche neben Kohlenstoffatomen 1 bis 4 Stickstoffatome bzw. 1 bis 3 Stickstoffatome als Ringglieder enthalten können, und in welchen 2 benachbarte Kohlenstoffringglieder oder ein Stickstoff- und ein benachbartes Kohlenstoffringglied durch eine Buta1,3-dien-1,4-diylgruppe verbrückt sein können, wobei diese Ringe über eines der Stickstoffringglieder an das Gerüst gebunden sind;
- 6-gliedriges Heteroaryl, enthaltend 1 bis 3 bzw. 1 bis 4 Stickstoffatome: 6-Ring-Heteroarylgruppen, welche neben Kohlenstoffatomen 1 bis 3 bzw. 1 bis 4 Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
- benzokondensiertes 6-gliedriges Heteroaryl, enthaltend 1 bis 4 Stickstoffatome: 6-Ring-Heteroarylgruppen, in welchen 2 benachbarte Kohlenstoffringglieder durch eine Buta-1,3-dien-1,4-diylgruppe verbrückt sein können, z.B. Chinolin, Isochinolin, Chinazolin und Chinoxalin.

Im Hinblick auf die fungizide Wirkung gegen Schadpilze wie z. B. *Pyricularia oryzae* sind Cycloalkylalkancarbonsäureamide I mit folgenden Substituenten bevorzugt, wobei die Bevorzugung jeweils für sich allein oder in Kombination zu sehen ist:

Das Kohlenstoffatom, welches die Gruppen R¹ und R² trägt, weist vorzugsweise R-Konfiguration auf.

Es sind Cycloalkylalkancarbonsäureamide I bevorzugt, bei denen R¹ Methyl und R² entweder Methyl oder Wasserstoff bedeutet; besonders bevorzugt sind Verbindungen I, bei denen R¹ für Methyl und R² für Wasserstoff steht.

Weiterhin sind Cycloalkylalkancarbonsäureamide der Formel I bevorzugt, in der W für gegebenenfalls substituiertes Phenyl steht, welches insbesondere in der 2-Position oder in den Positionen 2 und 4 substituiert ist. Ganz besonders bevorzugt ist die Substitution in 4-Stellung am Phenylring und bevorzugt dort die Substitution durch Cyano oder Methoxy, vorzugsweise durch Methyl und insbesondere durch Halogen, wobei hier wiederum Chlor bevorzugt ist.

Außerdem sind Cycloalkancarbonsäureamide der Formel I bevorzugt, in der W für 1- oder 2-Naphthyl steht, welches jeweils unsubstituiert oder durch ein bis drei der folgenden Gruppen substituiert ist: Halogen, Cyano, C₁-C₄-Alkyl oder C₁-C₄-Alkoxy. Besonders bevorzugt ist unsubstituiertes 1- oder 2-Naphthyl oder 2-Naphthyl, welches eine der folgenden Gruppen trägt: Chlor, Cyano, Methyl oder Methoxy. Insbesondere bevorzugt ist unsubstituiertes 2-Naphthyl.

Desweiteren sind Cycloalkylalkancarbonsäureamide I mit n = 1 bevorzugt. Die Substituenten R³ und R⁴ bedeuten vorzugsweise C₁-C₄-Alkyl und insbesondere Methyl oder Ethyl. Bevorzugt ist auch die Kombination, in der einer der beiden Substituenten für Wasserstoff und der jeweilig andere für C₁-C₄-Alkyl und insbesondere für Methyl oder Ethyl steht.

Ferner sind α-Chlor- oder α-Bromcycloalkylalkancarbonsäureamide I (Y = Brom oder Chlor) bevorzugt. Insbesondere sind α-Cyancycloalkylalkancarbonsäureamide I (Y = Cyan) bevorzugt.

Weiterhin sind Cycloalkylalkancarbonsäureamide der Formel I bevorzugt, in der A für ein substituiertes C₃-C₆-Cycloalkyl steht. Besonders bevorzugt ist ein methylierter C₃-C₆-Cycloalkylrest, der vorzugsweise den Methylsubstituenten am Kohlenstoffatom der Verknüpfungsstelle des Cycloalkanrings mit dem Restmolekül trägt.

Schließlich sind Cycloalkylalkancarbonsäureamide der Formel I bevorzugt, in der A für unsubstituiertes oder substituiertes Cyclopropyl steht. Insbesondere ist Cyclopropyl bevorzugt, welches einen bis drei Substituenten ausgewählt aus der Gruppe bestehend aus Chlor und C₁-C₃-Alkyl, insbesondere Methyl trägt. Chloriertes Cyclopropyl trägt vorzugsweise zwei Chloratome und diese in *geminaler* Stellung am Cyclopropanring. Alkyliertes oder bevorzugt methyliertes Cyclopropyl trägt vorzugsweise einen der Alkyl(Methyl)-substituenten am Kohlenstoffatom der Verknüpfungsstelle des Cyclopropanrings mit dem Restmolekül.

Insbesondere sind im Hinblick auf ihre Verwendung die in den an schließenden Tabellen zusammengestellten Verbindungen I bevorzugt.

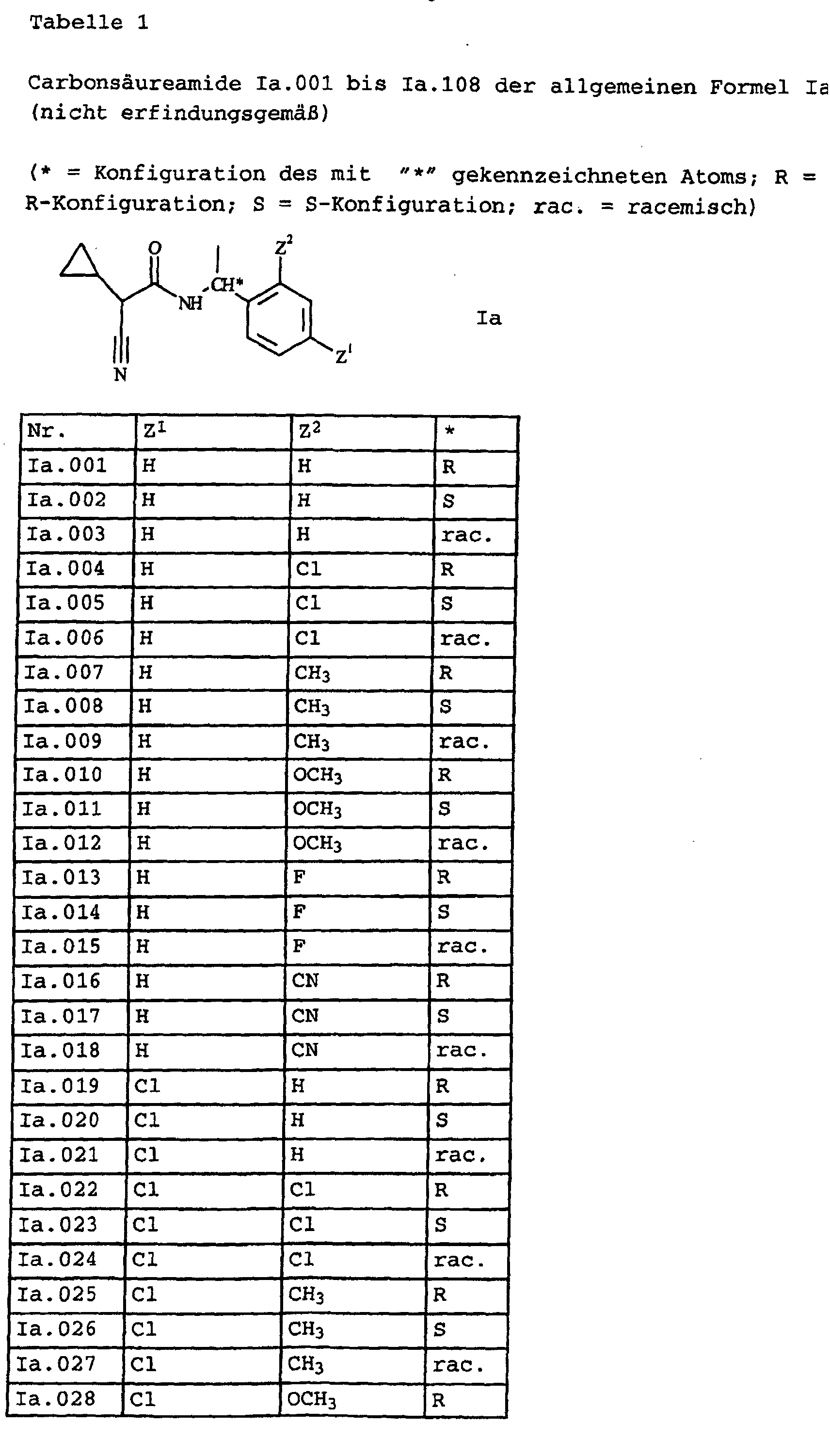

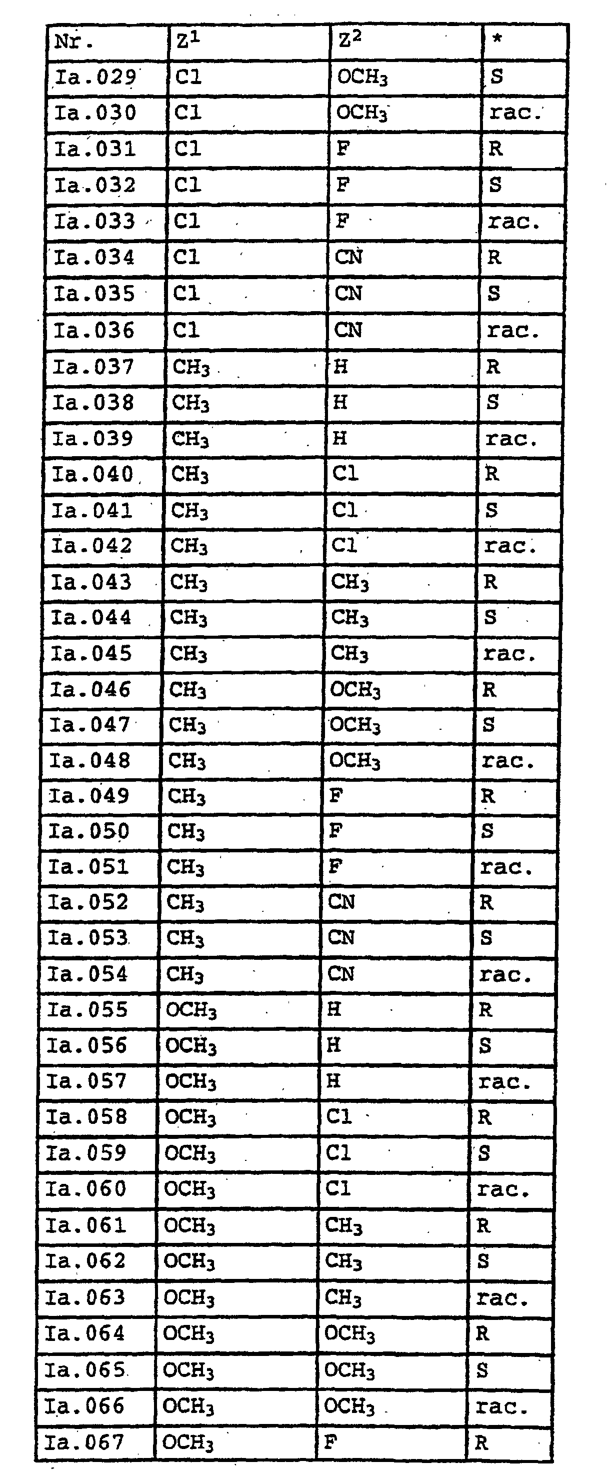

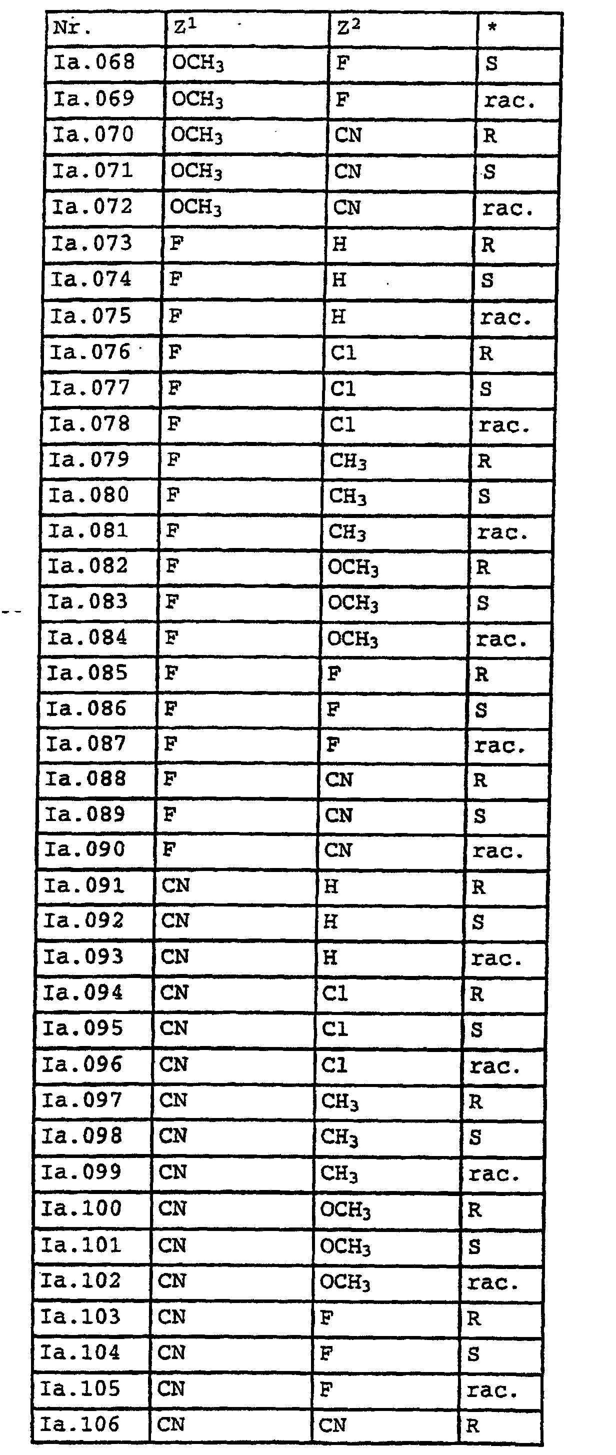

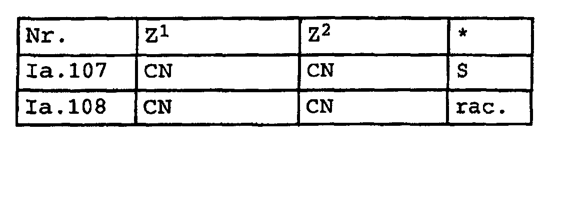

### Tabelle 2

Carbonsäureamide If.001 bis If.108 der allgemeinen Formel If, in welcher die Bedeutungen der Kombinationen aus Z¹, Z² und "*" durch die Zeilen der Tabelle 1 gegeben sind.

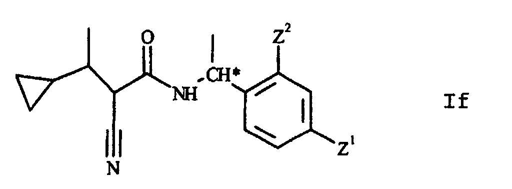

### Tabelle 3

Carbonsäureamide Ig.001 bis Ig.108 der allgemeinen Formel Ig, in welcher die Bedeutungen der Kombinationen aus Z¹, Z² und "*" durch die Zeilen der Tabelle 1 gegeben sind.

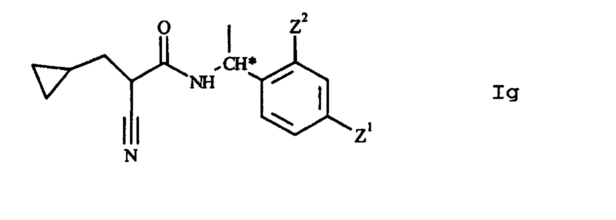

### Tabelle 4

Carbonsäureamide Ih.001 bis Ih.108 der allgemeinen Formel Ih, in welcher die Bedeutungen der Kombinationen aus Z¹, Z² und "*" durch die Zeilen der Tabelle 1 gegeben sind.

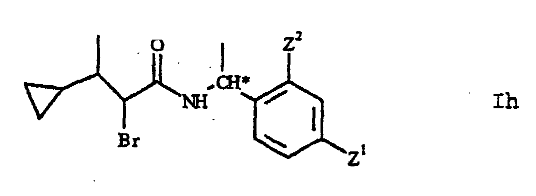

### Tabelle 5

Carbonsäureamide Ii.001 bis Ii.108 der allgemeinen Formel Ii, in welcher die Bedeutungen der Kombinationen aus Z¹, Z² und "*" durch die Zeilen der Tabelle 1 gegeben sind.

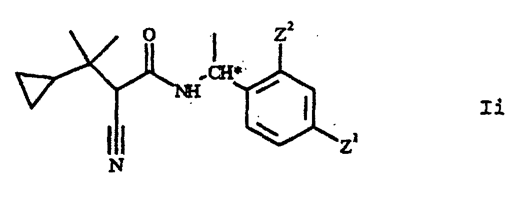

### Tabelle 6

Carbonsäureamide Ik.001 bis Ik.108 der allgemeinen Formel Ik, in welcher die Bedeutungen der Kombinationen aus Z¹, Z² und "*" durch die Zeilen der Tabelle 1 gegeben sind.

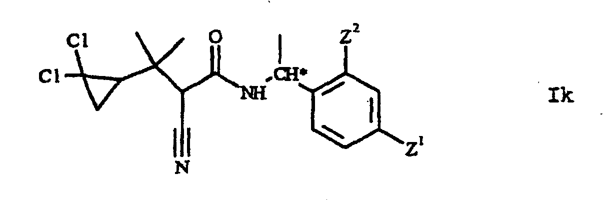

### Tabelle 7

Carbonsäureamide Im.001 bis Im.108 der allgemeinen Formel Im, in welcher die Bedeutungen der Kombinationen aus Z¹, Z² und "*" durch die Zeilen der Tabelle 1 gegeben sind.

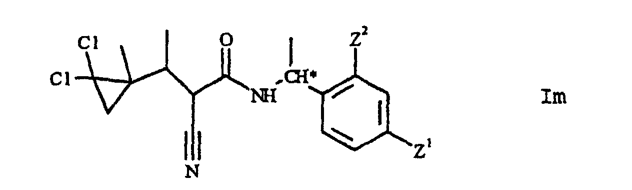

### Tabelle 8

Carbonsäureamide In.001 bis In.108 der allgemeinen Formel In, in welcher die Bedeutungen der Kombinationen aus Z¹, Z² und "*" durch die Zeilen der Tabelle 1 gegeben sind.

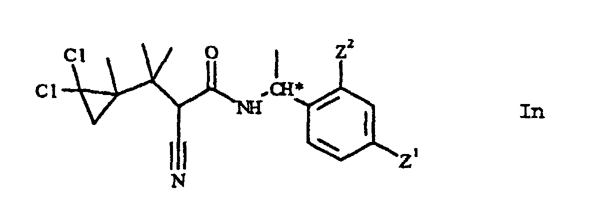

### Tabelle 9

Carbonsäureamide Io.001 bis Io.108 der allgemeinen Formel In, in welcher die Bedeutungen der Kombinationen aus Z¹, Z² und "*" durch die Zeilen der Tabelle 1 gegeben sind.

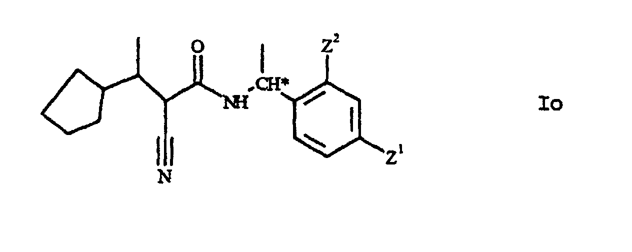

### Tabelle 10

Carbonsäureamide Ip.001 bis Ip.108 der allgemeinen Formel Ip, in welcher die Bedeutungen der Kombinationen aus Z¹, Z² und "*" durch die Zeilen der Tabelle 1 gegeben sind.

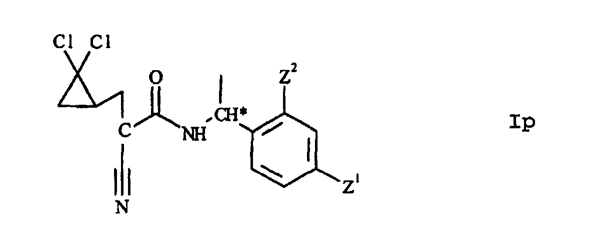

### Tabelle 11

Carbonsäureamide Ir.001 bis Ir.108 der allgemeinen Formel Ir, in welcher die Bedeutungen der Kombinationen aus Z¹, Z² und "*" durch die Zeilen der Tabelle 1 gegeben sind.

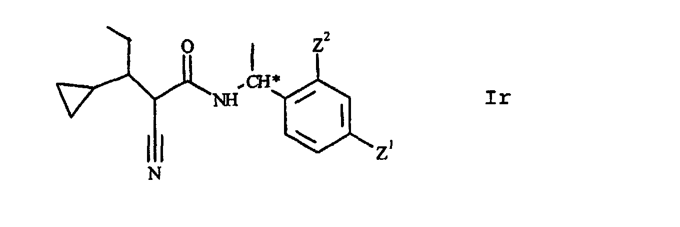

### Tabelle 12

Carbonsäureamide Is.001 bis Is.108 der allgemeinen Formel Is, in welcher die Bedeutungen der Kombinationen aus Z¹, Z² und "*" durch die Zeilen der Tabelle 1 gegeben sind.

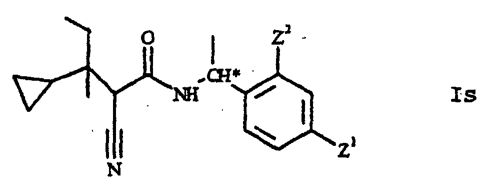

### Tabelle 13

Carbonsäureamide It.001 bis It.108 der allgemeinen Formel It, in welcher die Bedeutungen der Kombinationen aus Z¹, Z² und "*" durch die Zeilen der Tabelle 1 gegeben sind.

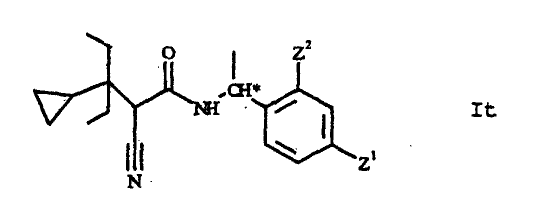

### Tabelle 14

Carbonsäureamide Iu.001 bis Iu.108 der allgemeinen Formel Iu, in welcher die Bedeutungen der Kombinationen aus Z¹, Z² und "*" durch die Zeilen der Tabelle 1 gegeben sind.

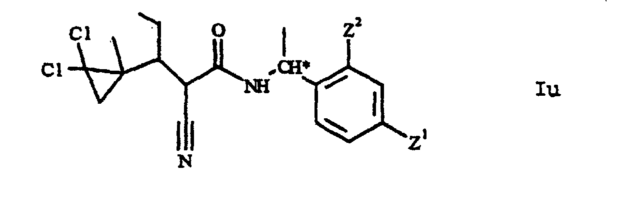

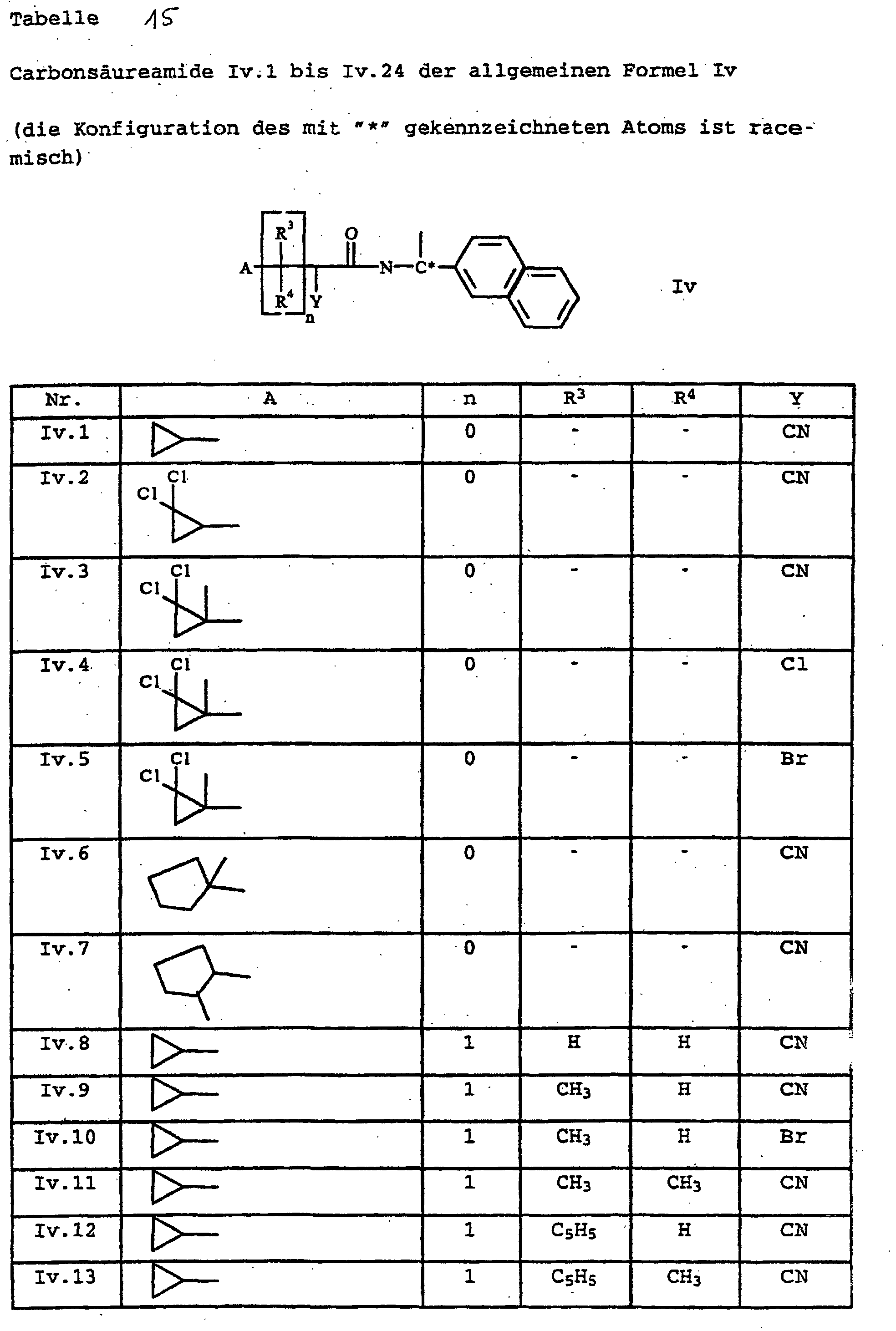

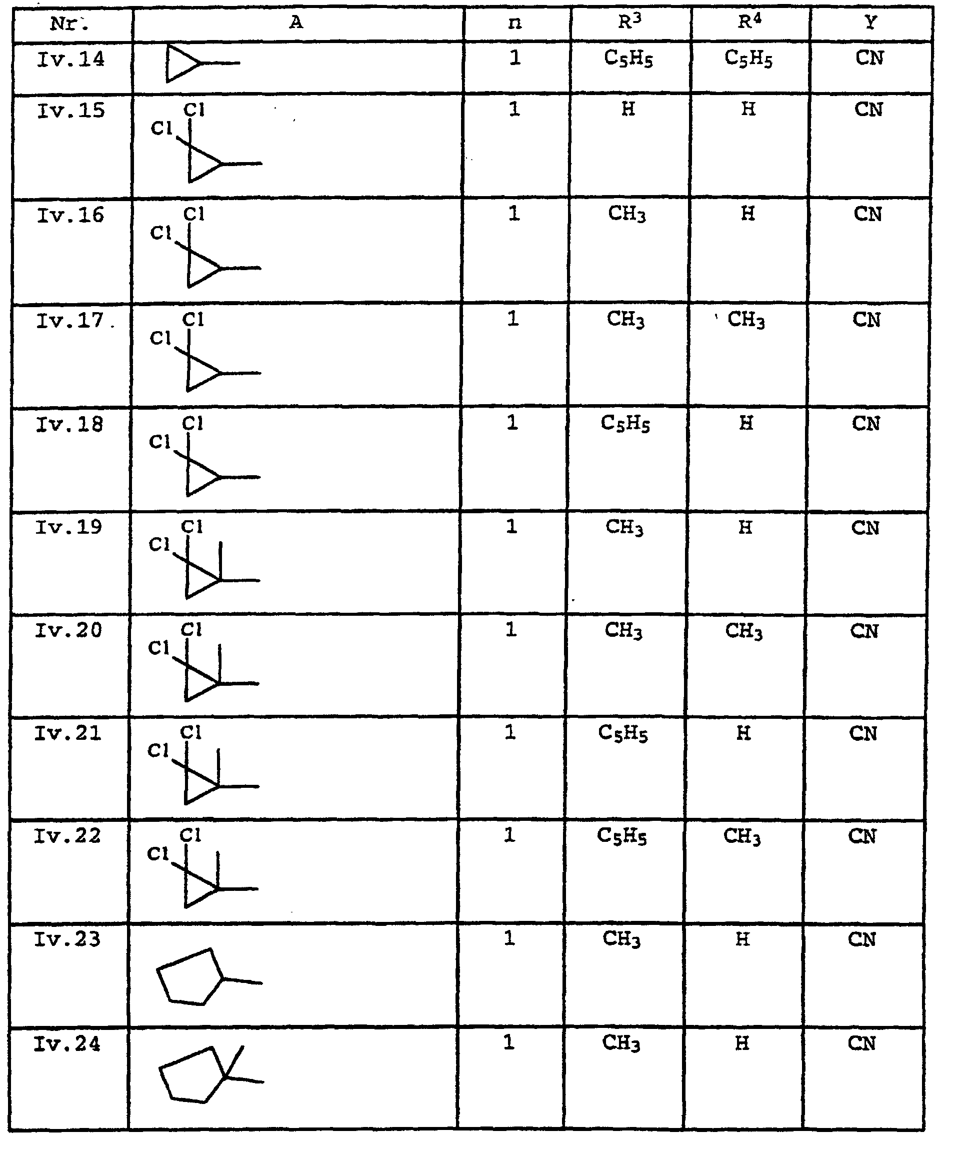

### Tabelle 16

Carbonsäureamide Iw.1 bis Iw.24 der allgemeinen Formel Iw, in welcher die Bedeutungen der Kombinationen aus A, n, R³, R⁴ und Y durch die Zeilen der Tabelle 16 gegeben sind.
(die Konfiguration des mit "*" gekennzeichneten Atoms ist R)

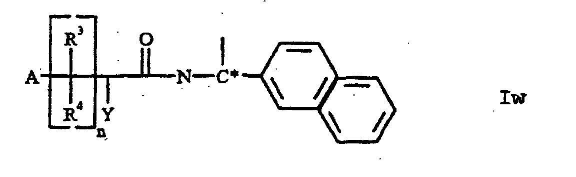

### Tabelle 17

Carbonsäureamide Iz.1 bis Iz.24 der allgemeinen Formel Iz, in welcher die Bedeutungen der Kombinationen aus A, n, R³, R⁴ und Y durch die Zeilen der Tabelle 16 gegeben sind.
(die Konfiguration des mit "*" gekennzeichneten Atoms ist racemisch)

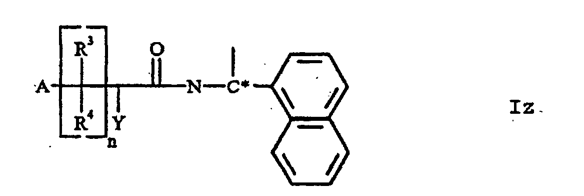

Nach einem erfindungsgemäß bevorzugten Verfahren werden die Carbonsäureamide I 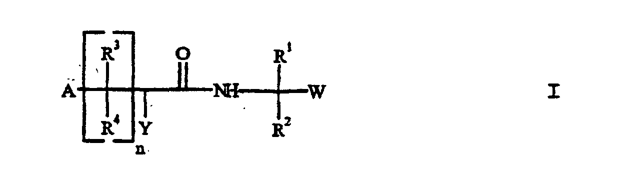 durch Umsetzung der Carbonsäurederivate II, 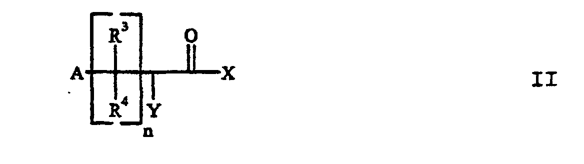 mit Aminen der Formel III 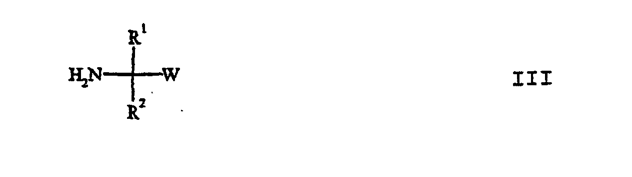 gewonnen.

Die Amidbildung erfolgt nach den literaturbekannten Verfahren. Dabei werden die freien Carbonsäuren der Formel II', wobei X für Hydroxy steht, in der Regel zuvor in eine aktiviertes Carbonsäurederivat II, wobei X z.B. für Chlor steht, übergeführt.

Die Aktivierung der Carbonsäuren II' kann vorzugsweise auch in situ erfolgen durch direkten Einsatz der Carbonsäuren II' unter Zusatz von z.B. Dicyclohexylcarbodiimid, Chlorameisensäureethylester, Cyanphosphonsäurediethylester, Triphenylphosphin/ Azodicarbonsäureester, 2-Pyridindisulfid/Triphenylphosphin, Carbonyldiimidazol, Thionylchlorid, Phosphortrichlorid, Phosphorpentachlorid, etc. Im allgemeinen erfolgt der Zusatz z.B. der Carbodiimide bezüglich der Carbonsäuren II' in äquimolaren Mengen.

Die Aktivierung der Carbonsäuren via Acylcyaniden geschieht beispielsweise durch Umsetzung der Carbonsäuren II' mit Cyanphosphonsäurediethylester, bevorzugt in einem inerten Lösungsmittel wie Tetrahydrofuran, Toluol oder Dichlormethan (vgl. Tetrahedron Lett. 18 (1973) 1595-8).

Die Aktivierung über Anhydride erfolgt beispielsweise durch Umsetzung der Carbonsäuren II' mit Kohlensäurechloriden wie Chlorameisensäureethylester im allgemeinen in Gegenwart von Basen und gegebenenfalls in einem inerten Lösungsmittel wie Toluol oder Tetrahydrofuran (vgl. "Houben-Weyl", 4. Aufl. (1974),15/1 Seite 28-32).

Die Amidbildung wird vorzugsweise in Gegenwart von Basen wie tertiären Aminen, z.B. Triethylamin oder Dimethylcyclohexylamin, Alkalimetallcarbonate, Alkalimetallhydroxide, Pyridin etc. durchgeführt. Die Reaktanden und die Hilfsbase werden zweckmäßigerweise in äquimolaren Mengen eingesetzt. Ein geringer Überschuß der Hilfsbase von 0,1 - 0,5 Äquivalenten kann unter Umständen hilfreich sein.

Als Lösungsmittel kommen aliphatische Kohlenwasserstoffe wie Hexan und Ligroin, aromatische Kohlenwasserstoffe wie Toluol und xylol, chlorierte Kohlenwasserstoffe wie Methylenchlorid und 1,2-Dichlorethan, Ether wie Methyl-tert-butylether und Tetrahydrofuran, polare aprotische Lösungsmittel, wie Acetonitril und Dimethylformamid oder Ester wie Essigsäureethylester oder Gemische hiervon in Frage.

Das Molverhältnis von Carbonsäurederivate II zu Amin III beträgt im allgemeinen 0.8 bis 1,5 und vorzugsweise 0,9 bis 1,1.

Nach vollständiger Umsetzung wird wie üblich aufgearbeitet, z.B. durch Eintrag der Reaktionsmischung in Wasser und anschließende Extraktion des Amids.

Die Amine der Formel III sind bekannt oder können leicht erhalten werden (vgl. Organikum (1993) Barth Verlagsgesellschaft mbH Leipzig, S. 509 ff; "Houben-Weyl", Band 15/1, Seiten 648-665; J. Am. Chem. Soc. 58, Seiten 1808-1811 (1936), Indian J. Chem. 10 (1972) 366)).

Aus Racematen der Amine III kann das R-Isomere in an sich bekannter Weise, etwa durch fraktionierte Kristallisation mit optisch aktiver Weinsäure oder vorzugsweise mittels enzymkatalysierter Veresterung und anschließender Verseifung separiert werden (vgl. z.B. die WO-A 95/08636).

Die Herstellung der α-Cyancyclopropanessigsäure ist in Org. Prep. Proced. Int. 5 (1973), 25-29 beschrieben. In Schema 1 wird eine generelle Route zum Aufbau von Carbonsäuren der Formel II' gegeben (vgl. Collect. Czech. Chem. Commun. 48 (1983) 1597-1601 und J. Polym. Sci., Polym. Chem. Ed. 14 (1976) 2357-9).

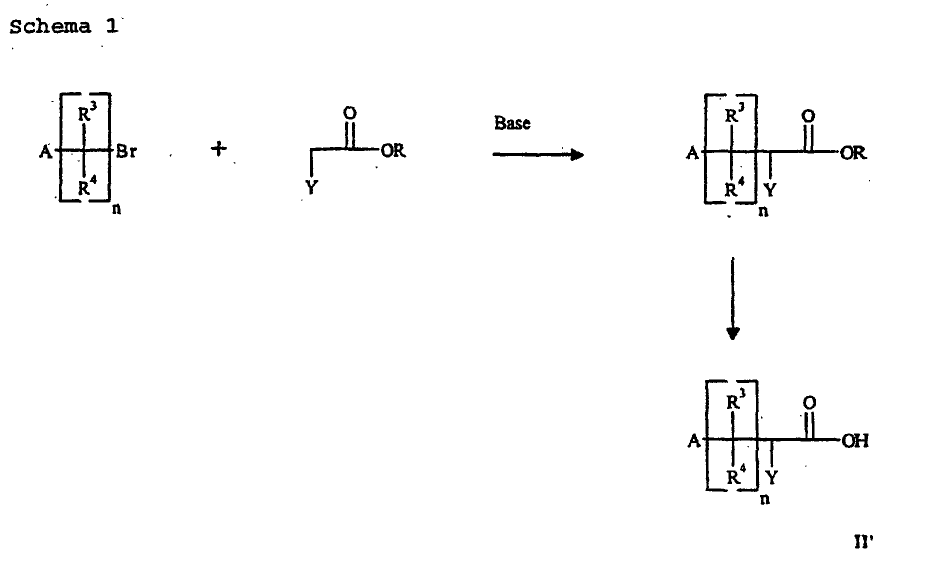

Carbonsäurederivate der Formel IIA lassen sich weiterhin gemäß Schema 2 herstellen.

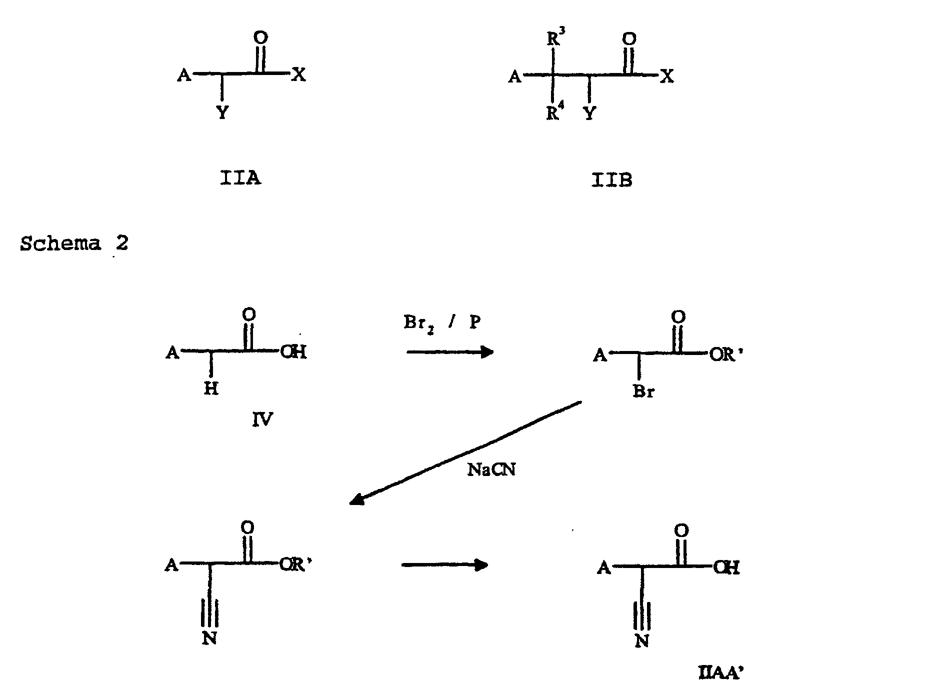

Die Cycloalkylessigsäuren der Formel IV, wobei A die in Anspruch 1 gegebene Bedeutung hat, sind bekannt (J. Chem. Technol. Biotechnol., Chem. Technol., 33A (1983) 109-15; NL 65 06 881; Chem. Ber. 41 (1908) 2627; Chem. Ber. 35 (1902) 2688).

Die Cycloalkylessigsäuren IV lassen sich gemäß der Vorschrift in J. Am. Chem. Soc 70 (1948) 3626 -7 α-ständig bromieren. Die Aufarbeitung in Gegenwart eines C₁-C₆-Alkohols führt unmittelbar zum entsprechenden Ester. Der anschließende Brom/Cyan-Austausch wird wie in Synth. Commun. 23 (1993) 2323-9 beschrieben durchgeführt. Die Hydrolyse der Ester zu den Carbonsäuren IIAA' erfolgt nach Standardverfahren (Organikum 1993 Barth Verlagsgesellschaft mbH, Leipzig, S. 431ff.).

Die Carbonsäurederivate der Formel IIB sind beispielsweise auf dem in Schema 3 gezeigten Weg zugänglich.

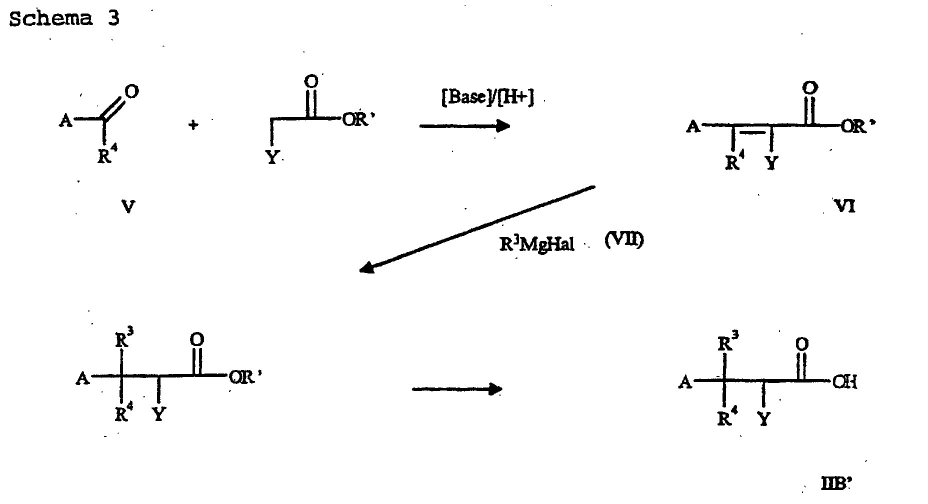

Die Ausgangsmaterialien, Acyl- oder Formylcycloalkane der Formel V, sind allgemein zugänglich (vgl. u.a. J. Chem. Soc, Perkin Trans. I, 6 (1994) 739 -52; EP-A 725 066). Diese werden in einer Knoevenagel Reaktion mit α-Halogen- oder α-Cyanessigsäure-C₁-C₆-alkylestern zu den Michael-Systemen VI umgesetzt (vgl. Chem. Heterocycl. Compd. 24 (1988) 860-4).

Die Kondensation wird üblicherweise in einem mit Wasser nicht mischbaren Lösungsmittel wie Hexan, Toluol oder Xylol unter Auskreisen des bei der Reaktion entstehenden Wassers durchgeführt. Dazu wird das Reaktionsgemisch mehrere Stunden unter Rückfluß zum Sieden erhitzt.

Als Katalysatoren dienen Basen wie z.B. Piperidin, Pyridin, Ammoniak oder β-Alanin in Gegenwart einer Säure wie beispielsweise Eisessig.

Anschließend addiert man eine Alkyl-Grignard-Verbindung der Formel VII, wobei R³ die in Anspruch 1 gegebene Bedeutung hat und Hal für Chlor, Brom oder Jod steht, an ein Michael-Systeme der Formel VI, um gesättigte Systeme vom Typ IIB zu erhalten.

Die Reaktion wird in unter Reaktionsbedingungen inerten Lösungsmitteln durchgeführt. Besonders bevorzugt sind Ether wie Tetrahydrofuran, Diethylether, Dimethoxyethan oder Methyltert.butylether. In der Regel wird eine Reaktionstemperatur von -10 bis 80°C und bevorzugt von 10 bis 60°C eingestellt.

Die Grignardverbindung VII wird in der Regel in äquimolaren Mengen bezogen auf das Michael-System VI eingesetzt. In manchen Fällen erweist es sich als vorteilhaft die Grignardverbindung in einem Überschuß von 0,2 bis 0,5 Moläquivalenten einzusetzen.

In der Regel wird die Addition kupferkatalysiert durch Zusatz von 1 - 10 Mol% von z.B. Kupfer(I)jodid durchgeführt. Man erreicht dadurch eine höhere Selektivität hinsichtlich 1,2-Addition versus 1,4-Addition.

Die freien Carbonsäuren IIB' schließlich werden durch alkalische Hydrolyse der entsprechenden Ester hergestellt (Organikum 1993 Barth Verlagsgesellschaft mbH, Leipzig, S. 431ff.).

Ein eleganter Zugang zur 2-Cyano-3-(2,2-dichlorcyclopropyl)-3-methylbutansäure wird in Schema 4 vorgestellt.

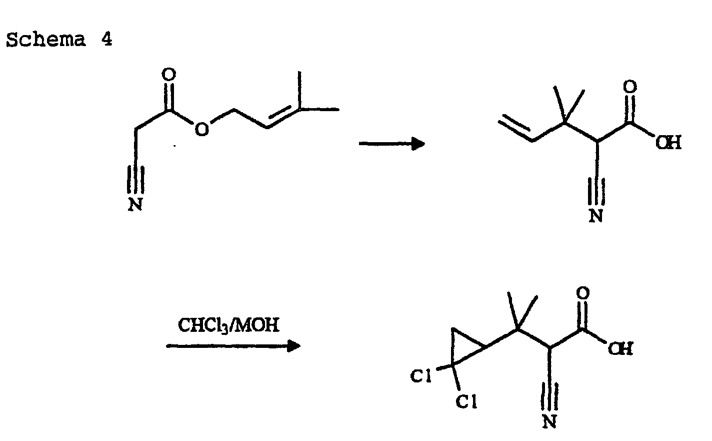

Die Herstellung von 2-Cyano-3,3-dimethylpent-4-ensäure aus dem 3-Methylbut-2-enylester der Cyanessigsäure ist in DE-A 26 49 711 sowie Res. Discl. (1985) 249,55 beschrieben. Durch Addition von Dichlorcarben, das aus Chloroform und Alkalimetallhydroxiden mittels Standardverfahren zugänglich ist, kann 2-Cyano-3-(2,2-dichlorcyclopropyl)-3-methylbutansäure direkt erhalten werden. Zur Steigerung der Ausbeute ist es zweckmäßig die Carbonsäürefunktion vor dem Cyclopropanierungsschritt zu schützen (beispielsweise durch Überführung in den tert.Butylester).

Durch die vorstehend genannten Verfahren sind Carbonsäurederivate II zugänglich, die sich beispielsweise zur Herstellung der erfindungsgemäßen Carbonsäureamide I eignen.

Die besonders bevorzugten Ausführungsformen der Carbonsäure-Derivate II im Hinblick auf die Substituenten R³, R⁴, A und Y entsprechen denjenigen der Carbonsäureamide I.

X steht für einen nukleophil austauschbaren Rest wie Hydroxy, C₁-C₄-Alkoxy, Halogen z. B. Brom oder Chlor, Hetaryl, z.B. Imidazolyl oder Pyridyl, Carboxylat, z.B. Acetat oder Trifluoracetat .

Insbesondere bevorzugt sind Carbonsäurederivate der Formel II, in der n 1 bedeutet und/oder A für gegebenenfalls substituiertes Cyclopropyl steht. Chloriertes Cyclopropyl trägt vorzugsweise zwei Chloratome und diese in *geminaler* Stellung am Cyclopropanring.

Die Verbindungen I zeichnen sich durch eine hervorragende Wirksamkeit gegen ein breites Spektrum von pflanzenpathogenen Pilzen, insbesondere aus der Klasse der Ascomyceten, Deuteromyceten, Phycomyceten und Basidiomyceten, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten: *Erysiphe graminis* (echter Mehltau) in Getreide, *Erysiphe cichoracearum und Sphaerotheca fuliginea* an Kürbisgewächsen, *Podosphaera leucotricha* an Äpfeln, *Uncinula necator* an Reben, *Puccinia*-Arten an Getreide, *Rhizoctonia*-Arten an Baumwolle, Reis und Rasen, *Ustilago*-Arten an Getreide und Zuckerrohr, *Venturia inaequalis* (Schorf) an Äpfeln, *Helminthosporium*-Arten an Getreide, *Septoria nodorum* an Weizen, *Botrytis cinerea* (Grauschimmel) an Erdbeeren, Gemüse, Zierpflanzen und Reben, *Cercospora arachidicola* an Erdnüssen, *Pseudocercosporella herpotrichoides* an Weizen, Gerste, *Pyricularia oryzae* an Reis, *Phytophthora infestans* an Kartoffeln und Tomaten, *Fusarium-* und *Verticillium*-Arken an verschiedenen Pflanzen, *Plasmopara viticola* an Reben, *Pseudoperonospera*-Arten an Hopfen und Gurken, *Alternaria*-Arten an Gemüse und Obst, sowie *Mycosphaerella*-Arten in Bananen.

Die Verbindungen I eignen sich außerdem zur Bekämpfung von Schadpilzen im Materialschutz (z.B. Holz, Papier, Dispersionen für den Ansprich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie-Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-diisopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid; 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecylmorpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis- (p-chlorphenyl)-3-pyridinmethanol, 5 1,2-Bis-(3-ethoxycarbonyl-2-thioureido)-benzol, 1,2-Bis-(3-methoxycarbonyl-2-thioureido)-benzol,
Strobilurine wie Methyl-E-methoxyimino-[α-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid,
Anilinopyrimidine wie N-(4,6-Dimethylpyrimidin-2-yl)-anilin, N-[4-Methyl-6-(1-propinyl) -pyrimidin-2-yl]-anilin, N- [4-Methyl-6-cyclopropyl-pyrimidin-2-yl]-anilin,
Phenylpyrrole wie 4-(2,2-Difluor-1,3-benzodioxol-4-yl)-pyrrol-3-carbonitril,
Zimtsäureamide wie 3-(4-Chlorphenyl)-3-(3,4-dimethoxyphenyl)-acrylsäuremorpholid,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die wirkstoffkonzentrationen in den anwendungsfertigen Zubereitungen können in größeren Bereichen variiert werden.

Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalinalpha-sulfonsäure, 10 Gew.-Teilen des Natriumsalzes einer Ligninsulfonsäure aus einer sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

### Herstellung der Carbonsäurederivate II

### Beispiel 1

### 2-Cyano-3-(2,2-dichlor-1-methylcyclopropyl)-butansäure (Verb. II.5 in Tabelle A)

a) 2-Cyano-3-(2,2-dichlor-1-methylcyclopropyl)-prop-2-ensäureethylester
   65,8 g (0,43 mol) (2,2-Dichlor-1-methylcylopropyl)carbaldehyd (vgl. hierzu J. Chem. Soc., Perkin Trans. I, Bd. 6 (1994) Seiten 739 - 52) und 48,6 g (0,43 mol) Cyanessigsäureethylester wurden analog Beispiel 3a) umgesetzt und ergaben nach Destillation 63 g (60 % Ausbeute) eines fahlgelben Öls.
   Sdp. 95 °C / 0,8 mbar, ¹H-NMR (in CDCl₃): 1,4 (t, 3H); 1,64 (s, 3H); 1,8 (d, 1H); 2.04 (d, 1H); 4,3 (q, 2H); 7,8 (s, 1H)).
b) 2-Cyano-3-(2,2-dichlor-1-methylcyclopropyl)-butansäureethylester
   5 g (20 mmol) des unter Beispiel 5a) erhaltenen Esters wurden analog Beispiel 3b) zu 5 g (95 % Ausbeute) 2-Cyano-3-(2,2-dichlor-1-methylcyclopropyl) -butansäureethylester umgesetzt. Sdp. 110°C / 0,5 mbar.
c) 2-Cyano-3-(2,2-dichlor-1-methylcyclopropyl)-butansäure.
   2 g (7,6 mmol) des unter Beispiel 5b) erhaltenen Esters wurden analog Beispiel 3c) zu 1,7 g (95% Ausbeute) der Säure als braunes Öl verseift.

### Beispiel 2

### 2-Cyano-3-(2,2-dichlorcyclopropyl)-3-methyl-butansäure (Verb. II.6 in Tabelle A)

2-Cyano-3,3-dimethyl-pent-4-ensäure (vgl. hierzu DE 2649711 und Res. Discl. (1985), 249, 55.) wurde gemäß der Vorschrift in J.Chem. Technol. Biotechnol., Chem. Technol. Bd. 33A, (1983), 109 - 15 cyclopropaniert.

### Beispiel 3

### 2-Cyano-3-(2,2-dichlorcyclopropyl)-propionsäure (Verb. II.7 in Tabelle A)

a) 2-Cyano-3-(2,2-dichlorcyclopropyl)-propionsäureethylester
   4,5 g (39 mmol) Cyanessigsäureethylester und 9,6 g (47 mmol) 1-Brom-1-(2,2-dichlorcyclopropyl)-methan wurden unter Stickstoff in 26 ml absolutem Ethanol vorgelegt. Dann wurden unter Rühren 7,0 g einer 30 %igen methanolischen Natriummethanolatlösung zugetropft. Es wurde für 2 Stunden am Rückfluß erhitzt. Nach dem Abkühlen auf Raumtemperatur wurde über Nacht gerührt. Nach dem Verdampfen des Methanols wurde unter Kühlung auf 10°C durch Zugabe von Wasser das ausgefallene NaBr vollständig gelöst. Die wäßrige Phase wurde dreimal mit Methyl-tert.-butylether extrahiert, dann wurden die vereinten organischen Phasen zweimal mit Wasser gewaschen. Nach dem Trocknen mit Natriumsulfat und Entfernen des Lösungsmittels wurde das als Rückstand erhaltene Rohprodukt fraktioniert destilliert. Es wurden 2,4 g (26 % Ausbeute)
   2-Cyano-3-(2,2-dichlorcyclopropyl)-propionsäureethylester erhalten.
b) 2-Cyano-3-(2,2-dichlorcyclopropyl)-propionsäure
   Die Verseifung des Esters gelang analog Beispiel 1c).

### Beispiel 4

### 2-Cyano-3-cyclopropylpentansäure (Verb. II.9 in Tabelle A)

a) 2-Cyano-3-cyclopropyl-pent-2-ensäuremethylester
   Eine Lösung aus 4 g (40,8 mmol) Cyclopropylethylketon und 2,4 g (24 mmol) Cyanessigsäuremethylester wurde analog Beispiel 3a) zu 0,9 g (21 % Ausbeute) 2-Cyano-3-cyclopropyl-pent-2-ensäuremethylester umgesetzt.
b) 2-Cyano-3-cyclopropylpentansäuremethylester
   1 g (5,6 mmol) des Esters 9a) wurden unter Stickstoff-Atmosphäre in 10 mL Tetrahydrofuran und 5 mL abs. Methanol vorgelegt. Nach Zugabe von 0,3 g (5,6 mmol) Kaliumborhydrid wurde bei Raumtemperatur über Nacht gerührt. Zur Aufarbeitung wurde mit Wasser hydrolysiert, mit 2n Salzsäure angesäuert und die wäßrige Phase mit Methyl-tert.-butylether extrahiert. Die vereinigten org. Phasen wurden mit Wasser und gesättigter Kochsalzlösung gewaschen. Nach dem Trocknen mit Natriumsulfat und Entfernen des Lösungsmittels wurden 0,6 g (60 % Ausbeute) 2-Cyano-3-cyclopropylpentansäuremethylester erhalten.
c) 2-Cyano-3-cyclopropylpentansäure
   0,6 g des Esters 9b) wurden analog Bsp. 1c) verseift. Es resultierten 0,54 g (98 % Ausbeute) der Titelverbindung.

### Beispiel 5

### 2-Cyano-3-cyclopropyl-3-methyl-pentansäure (Verb. II.10 in Tabelle A)

a) 2-Cyano-3-cyclopropyl-3-methyl-pentansäuremethylester
   1 g (5,6 mmol) des Esters 9a) wurden analog zu Beispiel 3b) umgesetzt. Es resultierten 1,1 g Rohprodukt, das ohne weitere Aufreinigung weiterverwendet wurde.
b) 2-Cyano-3-cyclopropyl-3-methyl-pentansäure
   0,5 g (2,56 mmol) des Esters 10a) wurden analog Bsp. 1c) verseift. Es resultierten 0,44 g (96 % Ausbeute) der Titelverbindung.

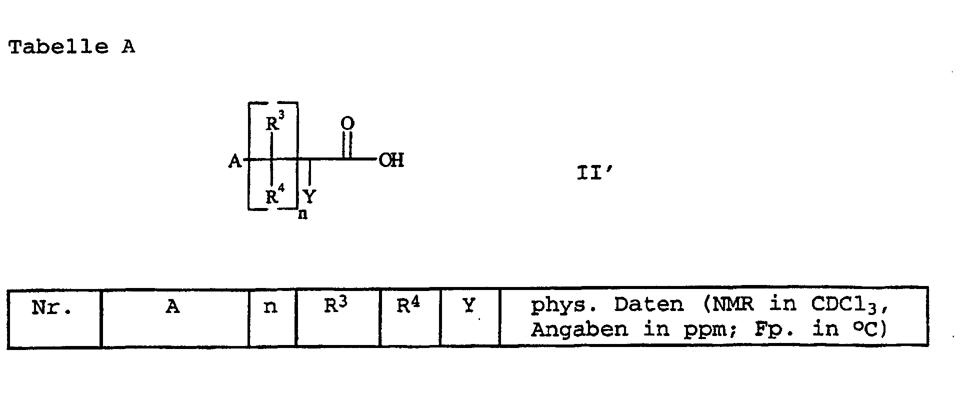

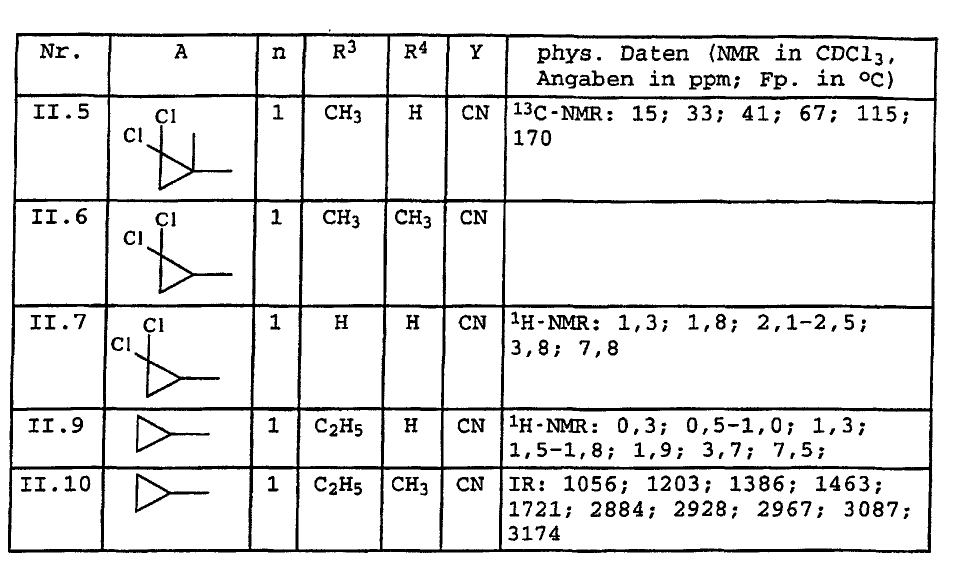

### Herstellung der Carbonsäureamide I

### Beispiel 6

### 2-cyano-3-cyclopropyl-butansäure-N-(4-chlorphenyl)-(R)-ethylamid (Verb. I.8 in Tabelle B)

Zu der Lösung von 0.46 g (3 mmol) 2-Cyano-3-cyclopropyl-butansäure und 0.47 g (3 mmol) R-1-(4-Chlorphenyl)ethylamin in 50 ml Dichlormethan wurden 3 mmol Triethylamin zugegeben. Sodann wurden 3 mmol 93 %-iger Cyanphosphonsäurediethylester bei 10°C zugetropft und 12 Stunden bei Raumtemperatur gerührt. Nach Zugabe von 50 ml Dichlormethan wurde mit 2N Natronlange, 5 %-iger Salzsäure und Wasser gewaschen. Anschließend wurde die organische Phase getrocknet und eingeengt. Der verbleibende Rückstand wurde chromatographisch an Kieselgel gereinigt. Als Rückstand verblieben 0.65 g (75% Ausbeute) der Titelverbindung als farbloses Harz.

### Beispiel 7

### 2-Cyano-3-cyclopropyl-3-methyl-butansäure-N-(4-chlorphenyl)-(R)-ethylamid (Verb. I.10 in Tabelle B)

Aus der Reaktion von 0,5 g (3mmol) 2-Cyano-3-cyclopropyl-3-methyl-butansäure mit 0,47 g (3 mmol) R-1-(4-Chlorphenyl)ethylamin erhielt man analog Beispiel 6 nach chromatographischer Reinigung 0,7 g (80 % Ausbeute) der Titelverbindung (Fp. 103 - 6°C).

### Beispiel 8

### 2-Cyano-3-(2,2-dichlor-1-methylcyclopropyl)-butansäure-N-(4-chlorphenyl)-(R)-ethylamid (Verb. I.12 in Tabelle B)

Aus der Reaktion von 2 g (8.5 mmol) 2-Cyano-3-(2,2-dichlor-1-methylcyclopropyl)-butansäure mit 1.3 g (8.5 mmol) R-1-(4-Chlorphenyl)ethylamin erhielt man analog Beispiel 6 die 3.1 g der Titelverbindung (farbloses Harz) als Diastereomerengemisch.

### Beispiel 9

### 2-Cyano-3-(2,2-dichlorcyclopropyl)-3-methyl-butansäure-N-(4-chlorphenyl)-(R)-ethylamid (Verb. I.15 in Tabelle B)

Die Umsetzung der unter Beispiel 6 erhaltenen Säure mit R-1-(4-Chlorphenyl)ethylamin ergab analog Beispiel 6 die Titelverbindung als Diastereomerengemisch.

In Tabelle B sind weitere Cycloalkylalkancarbonsäureamide I aufgeführt, die auf die gleiche Weise hergestellt wurden oder herstellbar sind:

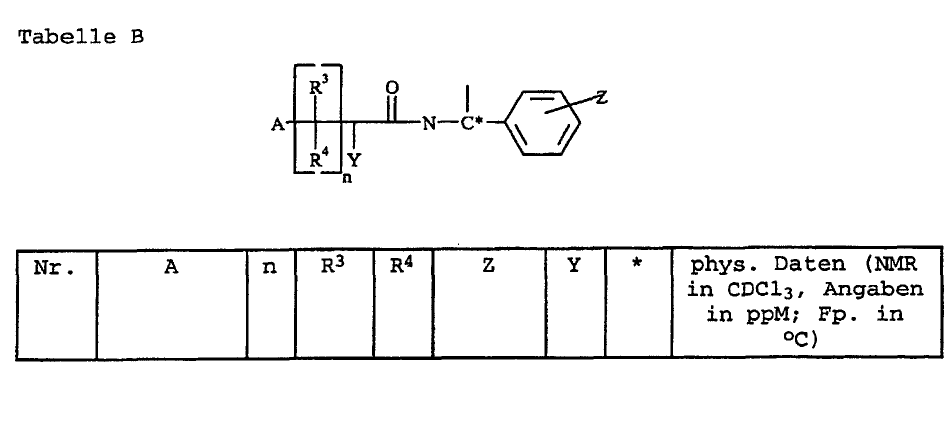

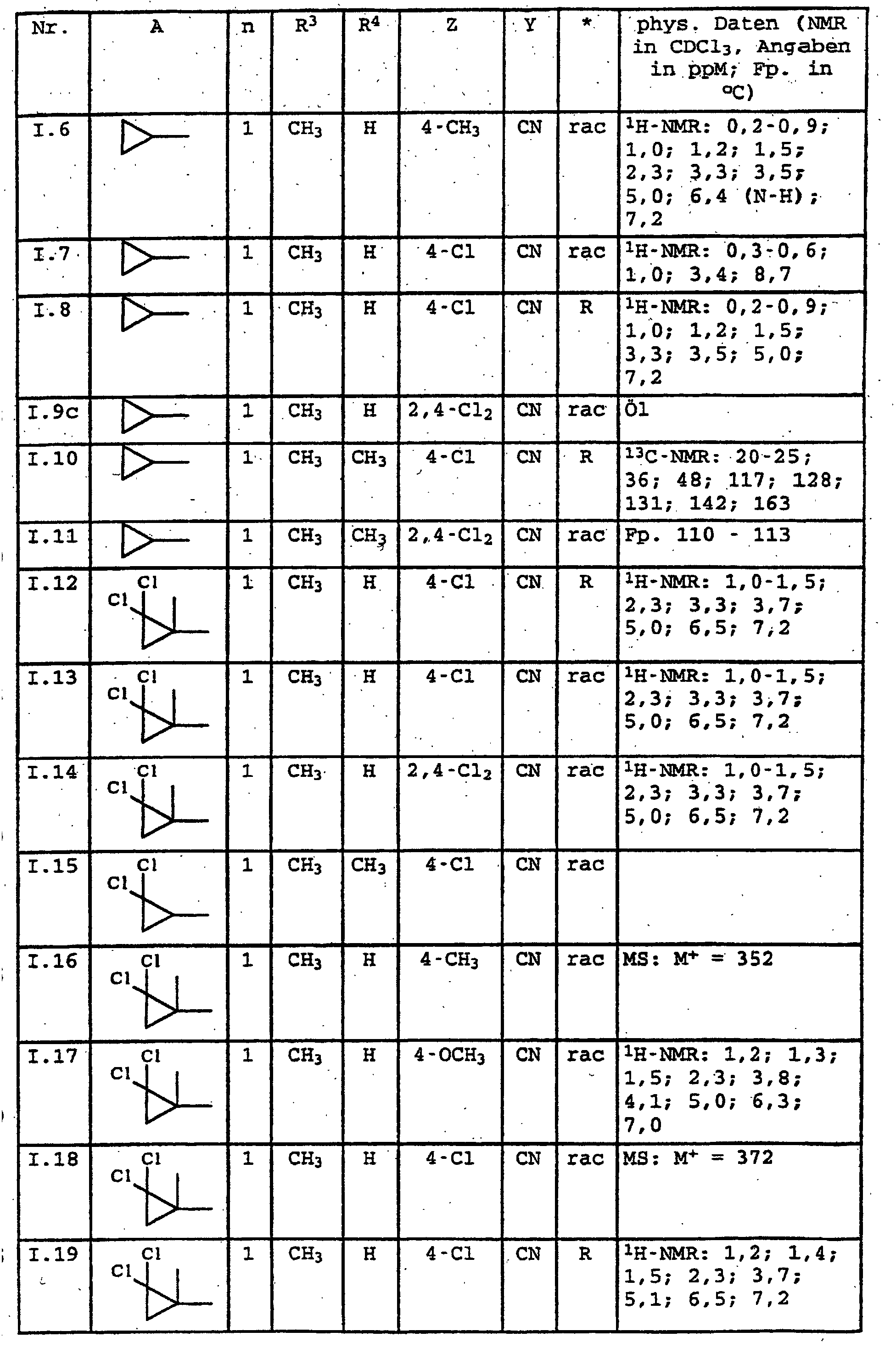

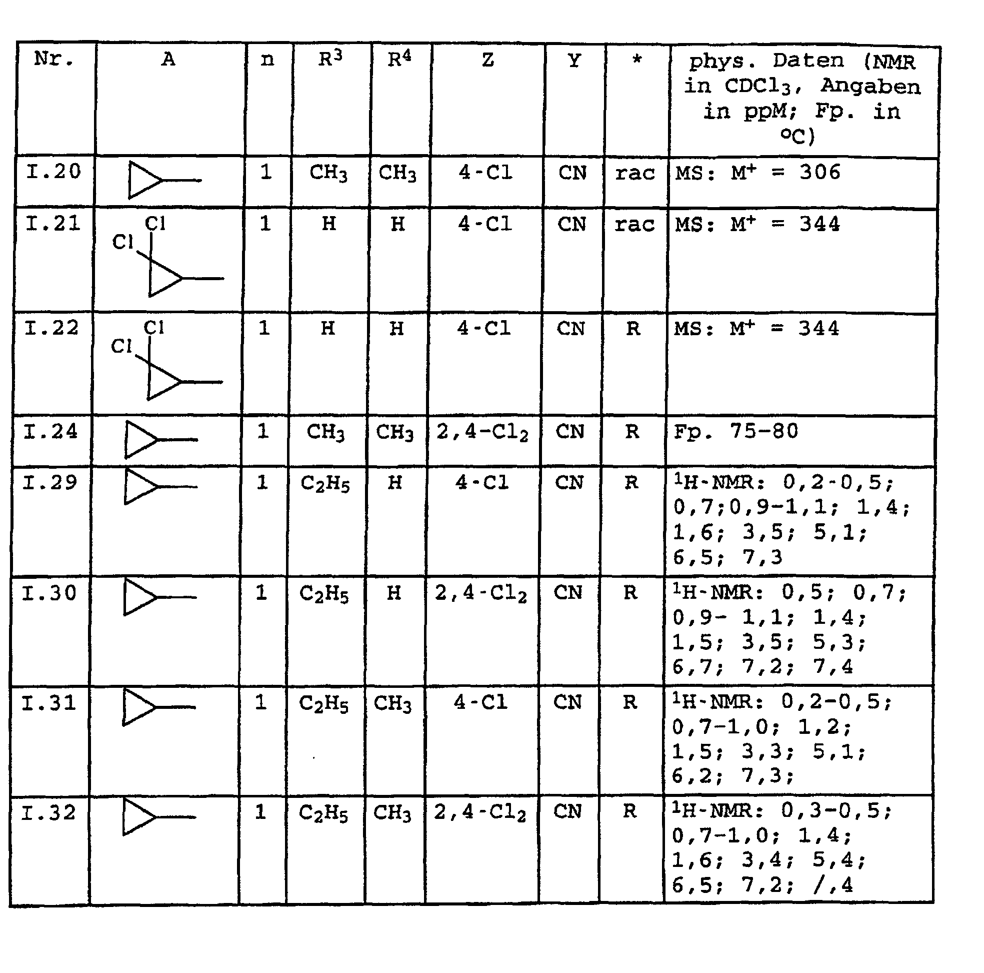

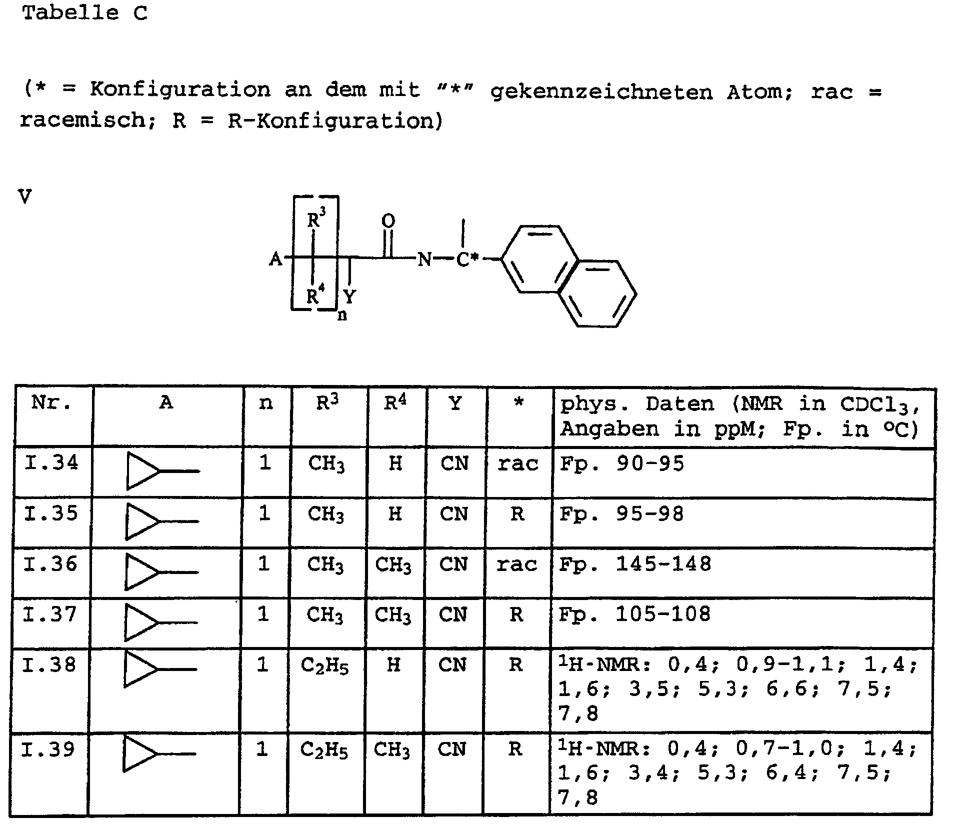

### Anwendungsbeispiele

Die fungizide Wirkung der Verbindungen der allgemeinen Formel I gegen Schadpilze ließ sich durch folgende Gewächshausversuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor®EL (Emulan® EL, Emulgator auf der Basis ethoxylierter Fettalkohole) aufbereitet und entsprechend der gewünschten Konzentration mit Wasser verdünnt.
1. Wirksamkeit gegen *Pyricularia oryzae* (protektiv)
   Blätter von in Töpfen gewachsenen Reiskeimlingen (Sorte "Tai-Nong 67") wurden mit der wäßrigen Aufbereitung der Wirkstoffe (250 ppm-haltig) behandelt. Nach ca. 24 Stunden wurden die Pflanzen mit einer wäßrigen Sporensuspension von *Pyricularia oryzae* inokuliert. Die so behandelten Pflanzen wurden in Klimakammern bei 22 - 24°C und 95 bis 99% relativer Luftfeuchte für 6 Tage aufgestellt. Anschließend wurde das Ausmaß der Befallsentwicklung auf den Blättern visuell ermittelt.
   In diesem Test zeigten die mit den erfindungsgemäßen Verbindungen I.7, I.8 und I.10 behandelten Pflanzen einen Befall von 10% und weniger und die unbehandelten Pflanzen 80 % Befall.
2. Systemische Wirksamkeit gegen *Pyricularia oryzae*
   Vorgekeimter Reis (Sorte "Tai-Nong 67") wurde in einer Hydrokultur mit Hoagland-Lösung bis zum Zweiblattstadium kultiviert. Dann wurden die Wurzeln mit der wäßrigen Aufbereitung der Wirkstoffe (50 ppm-haltig) angegossen. Nach fünf Tagen weiterer Kultivierung im Gewächshaus wurden die Pflanzen mit einer wäßrigen Sporensuspension von *Pyricularia oryzae* inokuliert. Die so behandelten Pflanzen wurden in Klimakammern bei 22 - 24°C und 95 bis 99% relativer Luftfeuchte für 6 Tage aufgestellt. Anschließend wurde das Ausmaß der Befallsentwicklung auf den Blättern visuell ermittelt.
   In diesem Test zeigten die mit den erfindungsgemäßen Verbindungen I.7, I.8 und I.10 behandelten Pflanzen einen Befall von 15% und weniger und die unbehandelten Pflanzen 80% Befall.

## Patentansprüche

1. Cycloalkylalkancarbonsäureamide der allgemeinen Formel I 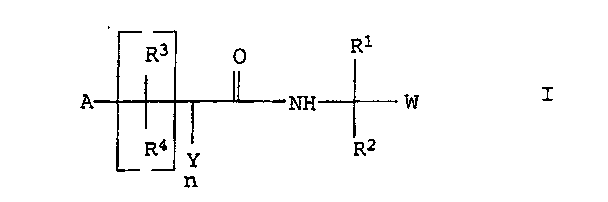 in der die Substituenten die folgende Bedeutung haben:
A C₃-C₆-Cycloalkyl, welches einen bis drei Substituenten ausgewählt aus der Gruppe bestehend aus Halogen und C₁-C₃-Alkyl tragen kann;
R¹ C₁-C₆-Alkyl oder C₂-C₆-Alkenyl, wobei diese Reste partiell oder vollständig halogeniert sein und/oder eine oder zwei der folgenden Gruppen tragen können: C₁-C₄-Alkoxy, C₁-C₄-Halogenalkoxy, C₁-C₄-Alkylthio, C₁-C₄-Alkoxycarbonyl, C₃-C₆-Cycloalkyl und Phenyl, wobei das Phenyl partiell oder vollständig halogeniert sein und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy, C₁-C₄-Alkylthio, C₃-C₆-Cycloalkyl oder Heterocyclyl;
R²,R³,R⁴ Wasserstoff oder unabhängig von jener eine der Bedeutungen des Restes R¹;
n 1;
Y Cyano oder Halogen;
W Phenyl, welches eine bis drei der folgenden Gruppen tragen kann: Nitro, Halogen, Cyano, C₁-C₄-Alkyl, C₁-C₄-Halogenalkyl, C₁-C₄-Alkoxy, C₁-C₄-Halogenalkoxy, C₁-C₄-Alkylthio, C₃-C₆-Cycloalkyl und C₁-C₄-Alkoxycarbonyl.

2. Cycloalkylalkancarbonsäureamide der allgemeinen Formel I 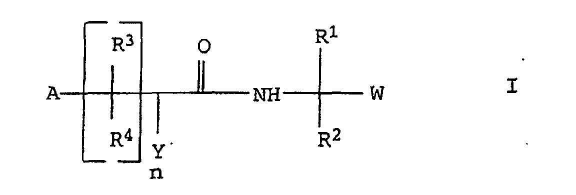 in der die Substituenten die folgende Bedeutung haben:
A C₃-C₆-Cycloalkyl, welches einen bis drei Substituenten ausgewählt aus der Gruppe bestehend aus Halogen und C₁-C₃-Alkyl tragen kann;
R¹ C₁-C₆-Alkyl oder C₂-C₆-Alkenyl, wobei diese Reste partiell oder vollständig halogeniert sein und/oder eine oder zwei der folgenden Gruppen tragen können: C₁-C₄-Alkoxy, C₁-C₄-Halogenalkoxy, C₁-C₄-Alkylthio, C₁-C₄-Alkoxycarbonyl, C₃-C₆-Cycloalkyl und Phenyl, wobei das Phenyl partiell oder vollständig halogeniert sein und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy, C₁-C₄-Alkylthio, C₃-C₆-Cycloalkyl oder Heterocyclyl;
R²,R³,R⁴ Wasserstoff oder unabhängig von jener eine der Bedeutungen des Restes R¹;
n 0 oder 1;
Y Cyano oder Halogen;
W Naphthyl oder Heteroaryl, wobei diese Reste eine bis drei der folgenden Gruppen tragen können: Nitro, Halogen, Cyano, C₁-C₄-Alkyl, C₁-C₄-Halogenalkyl, C₁-C₄-Alkoxy, C₁-C₄-Halogenalkoxy, C₁-C₄-Alkylthio, C₃-C₆-Cycloalkyl und C₁-C₄-Alkoxycarbonyl,
ausgenommen von Verbindungen der Formel I , wobei
n 0 und
A Cyclopentyl ist.

3. Cycloalkylalkancarbonsäureamide der allgemeinen Formel I gemäß Anspruch 1 oder 2, in der
A Cyclopropyl, welches einen bis drei Substituenten ausgewählt aus der Gruppe bestehend aus Halogen und C₁-C₃-Alkyl tragen kann,
bedeutet.

4. Cycloalkylalkancarbonsäureamide der allgemeinen Formel I gemäß einem der Ansprüche 1 bis 3, wobei A Cycloproyl bedeutet, das durch zwei Chloratome substituiert ist.

5. Cycloalkylalkancarbonsäureamide der allgemeinen Formel I gemäß einem der Anspruch 1 oder 2 - 4, wobei A Cyclopropyl bedeutet, das durch eine Alkylgruppe am Kohlenstoffatom der Verknüpfungsstelle des Cyclopropanrings mit dem Restmolekül substituiert ist.

6. Cycloalkylalkancarbonsäureamide der allgemeinen Formel I gemäß einem der Ansprüche 1 - 5, in der
R¹ Methyl und
R², R³,R⁴ Wasserstoff oder Methyl
bedeuten.

7. Cycloalkylalkancarbonsäureamide der allgemeinen Formel I gemäß Anspruch 2, in der
n 1
bedeutet.

8. Verfahren zur Herstellung von Cycloalkylalkancarbonsäureamiden der allgemeinen Formel I gemäß einem der Ansprüche 1 - 7, 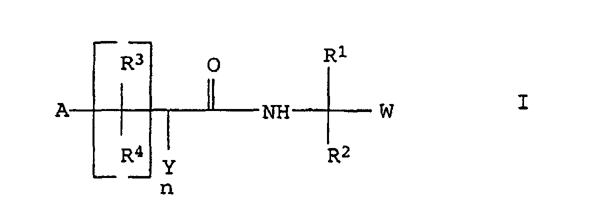 **dadurch gekennzeichnet, daß** ein Carbonsäurederivat der Formel II, 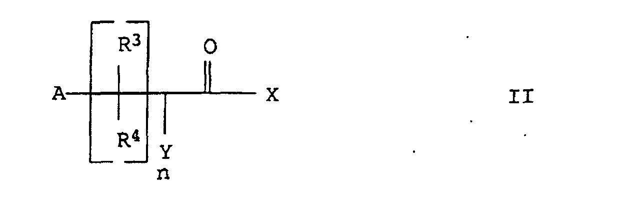 in der die Reste A, Y, R³ und R⁴ sowie n die in Anspruch 1 oder 2 angegebene Bedeutung haben und X für einen nukleophil austauschbaren Rest steht,
mit einem Amin der Formel III, 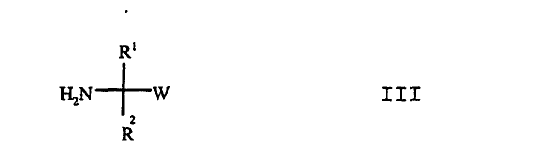 in der die Reste W, R¹ und R² die in Anspruch 1 genannte Bedeutung besitzen, zur Reaktion gebracht wird.

9. Carbonsäure-Derivate der Formel IIB, 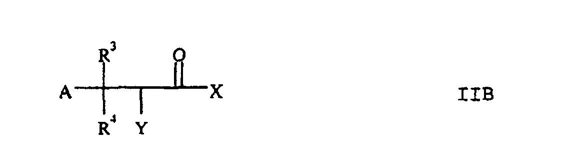 in der
X für einen nukleophil austauschbaren Rest steht;
A C₃-C₆-Cycloalkyl bedeutet, welches durch einen bis drei Substituenten ausgewählt aus der Gruppe bestehend aus Halogen und C₁-C₃-Alkyl substituiert ist,
und die Reste R³, R⁴ und Y die in Anspruch 1 gegebene Bedeutung besitzen.

10. Carbonsäure-Derivate gemäß Anspruch 9, wobei A Cyclopropyl bedeutet, das durch zwei Chloratome substituiert ist.

11. Carbonsäure-Derivate gemäß einem der Ansprüche 9 bis 10, wobei A Cyclopropyl bedeutet, das durch eine Alkylgruppe am Kohlenstoffatom der Verknüpfungsstelle des Cyclopropanrings mit dem Restmolekül substituiert ist.

12. Mittel, enthaltend eine zur Bekämpfung von Schadpilzen wirksame Menge mindestens eines Cycloalkylalkancarbonsäureamids der Formel I gemäß einem der Ansprüche 1 bis 7 und mindestens einen inerten flüssigen und/oder festen Trägerstoff sowie gegebenenfalls mindestens einen oberflächenaktiven Stoff.

13. Verfahren zur Bekämpfung von Schadpilzen, **dadurch gekennzeichnet, daß** man die Schadpilze, deren Lebensraum oder die von ihnen freizuhaltenden Pflanzen, Flächen, Materialien oder Räume mit einer wirksamen Menge eines Cycloalkylalkancarbonsäureamids der Formel I gemäß einem der Ansprüche 1 - 7 behandelt.

14. Verwendung eines Cycloalkylalkancarbonsäureamids der allgemeinen Formel I gemäß einem der Ansprüche 1 - 7 zur Bekämpfung von Schadpilzen.

## Claims

1. A cycloalkylalkanecarboxamide of the formula I 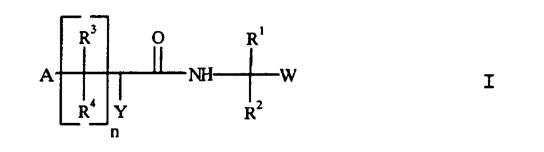 where the substituents have the following meanings:
A is C₃-C₆-cycloalkyl which can have attached to it one to three substituents selected from the group consisting of halogen and C₁-C₃-alkyl;
R¹ is C₁-C₆-alkyl or C₂-C₆-alkenyl, it being possible for these radicals to be partially or fully halogenated and/or to have attached to them one or two of the following groups: C₁-C₄-alkoxy, C₁-C₄-haloalkoxy, C₁-C₄-alkylthio, C₁-C₄-alkoxycarbonyl, C₃-C₆-cycloalkyl and phenyl, it being possible for the phenyl to be partially or fully halogenated and/or have attached to it one to three of the following radicals: nitro, cyano, C₁-C₄-alkyl, C₁-C₄-haloalkyl, C₁-C₄-alkoxy, C₁-C₄-haloalkoxy, C₁-C₄-alkylthio, C₃-C₆-cycloalkyl or heterocyclyl;
R²,R³ and R⁴ are hydrogen or, independently of this meaning, have one of the meanings of the radical R¹;
n is 1;
Y is cyano or halogen;
W is phenyl which can have attached to it one to three of the following groups:
nitro, halogen, cyano, C₁-C₄-alkyl, C₁-C₄-haloalkyl, C₁-C₄-alkoxy, C₁-C₄-haloalkoxy, C₁-C₄-alkylthio, C₃-C₆-cycloalkyl and C₁-C₄-alkoxycarbonyl.

2. A cycloalkylalkanecarboxamide of the formula I 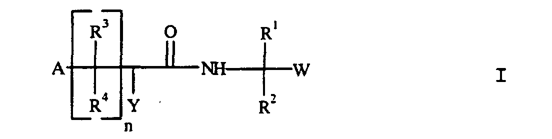 where the substituents have the following meanings:
A is C₃-C₆-cycloalkyl which can have attached to it one to three substituents selected from the group consisting of halogen and C₁-C₃-alkyl;
R¹ is C₁-C₆-alkyl or C₂-C₆-alkenyl, it being possible for these radicals to be partially or fully halogenated and/or to have attached to them one or two of the following groups: C₁-C₄-alkoxy, C₁-C₄-haloalkoxy, C₁-C₄-alkylthio, C₁-C₄-alkoxycarbonyl, C₃-C₆-cycloalkyl and phenyl, it being possible for the phenyl to be partially or fully halogenated and/or have attached to it one to three of the following radicals: nitro, cyano, C₁-C₄-alkyl, C₁-C₄-haloalkyl, C₁-C₄-alkoxy, C₁-C₄-haloalkoxy, C₁-C₄-alkylthio, C₃-C₆-cycloalkyl or heterocyclyl;
R², R³ and R⁴ are hydrogen or, independently of this meaning, have one of the meanings of the radical R¹;
n is 0 or 1;
Y is cyano or halogen;
W is naphthyl or heteroaryl, it being possible for these radicals to have attached to them one to three of the following groups: nitro, halogen, cyano, C₁-C₄-alkyl, C₁-C₄-haloalkyl, C₁-C₄-alkoxy, C₁-C₄-haloalkoxy, C₁-C₄-alkylthio, C₃-C₆-cycloalkyl and C₁-C₄-alkoxycarbonyl,
with the exception of the compounds of the formula I where
n is 0 and
A is cyclopentyl.

3. A cycloalkylalkanecarboxamide of the formula I as claimed in claim 1 or 2 where
A is cyclopropyl which can have attached to it one to three substituents selected from the group consisting of halogen and C₁-C₃-alkyl.

4. A cycloalkylalkanecarboxamide of the formula I as claimed in any of claims 1 to 3 where A is cyclopropyl which is substituted by two chlorine atoms.

5. A cycloalkylalkanecarboxamide of the formula I as claimed in any of claims 1 or 2 - 4 where A is cyclopropyl which is substituted by an alkyl group on the carbon atom of the position at which the cylopropane ring is attached to the remainder of the molecule.

6. A cycloalkylalkanecarboxamide of the formula I as claimed in any one of claims 1 - 5 where
R¹ is methyl and
R², R³ and R⁴ are hydrogen or methyl.

7. A cycloalkylalkanecarboxamide of the formula I as claimed in claim 2 where
n is 1.

8. A process for the preparation of a cycloalkylalkanecarboxamide of the formula I as claimed in any one of claims 1 - 7, 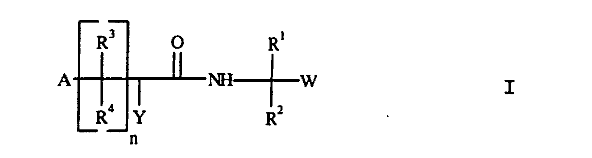 which comprises reacting a carboxylic acid derivative of the formula II 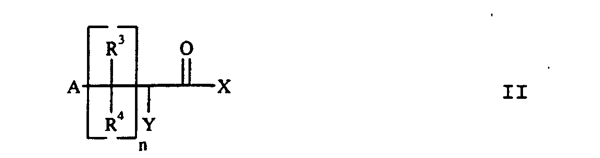 where the radicals A, Y, R³ and R⁴ and also n have the meanings given in claim 1 or 2 and X is a nucleophilically exchangeable radical
with an amine of the formula III 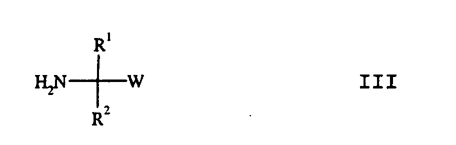 where the radicals W, R¹ and R² have the meanings given in claim 1.

9. A carboxylic acid derivative of the formula IIB 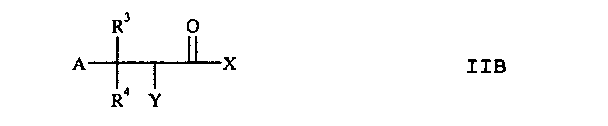 where
X is a nucleophilically exchangeable radical;
A is C₃-C₆-cycloalkyl which is substituted by one to three substituents selected from the group consisting of halogen and C₁-C₃-alkyl,
and the radicals R³, R⁴ and Y have the meanings given in claim 1.

10. A carboxylic acid derivative as claimed in claim 9 where A is cyclopropyl which is substituted by two chlorine atoms.

11. A carboxylic acid derivative as claimed in claim 9 or 10 where A is cyclopropyl which is substituted by an alkyl group on the carbon atom of the position at which the cyclopropane ring is attached to the remainder of the molecule.

12. A composition comprising such an amount of at least one cycloalkylalkanecarboxamide of the formula I as claimed in any one of claims 1 - 7 that it is effective for controlling harmful fungi and at least one inert liquid and/or solid carrier and, if appropriate, at least one surfactant.

13. A method of controlling harmful fungi, which comprises treating the harmful fungi, their environment or the plants, areas, materials or spaces to be kept free from them with an effective amount of a cycloalkylalkanecarboxamide of the formula I as claimed in any one of claims 1 - 7.

14. The use of a cycloalkylalkanecarboxamide of the formula I as claimed in any one of claims 1 - 7 for controlling harmful fungi.

## Revendications

1. Amides d'acide cycloalkylalcanecarboxylique de formule générale I 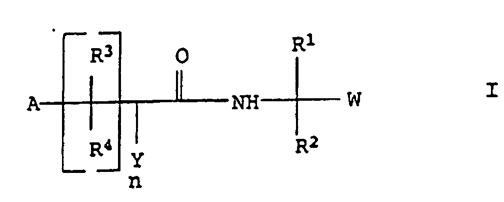 où les substituants ont la signification suivante:
A cycloalkyle en C₃-C₆, qui peut porter un à trois substituants choisis dans le groupe consistant en halogéno et alkyle en C₁-C₃;
R¹ alkyle en C₁-C₆ ou alcényle en C₂-C₆, tandis que ces restes sont partiellement ou totalement halogénés et/ou peuvent porter un ou deux des groupes suivants: alcoxy en C₁-C₄, halogénoalcoxy en C₁-C₄, alkylthio en C₁-C₄, alcoxy(C₁-C₄)carbonyle, cycloalkyle en C₃-C₆ et phényle, tandis que le phényle est partiellement ou totalement halogéné et/ou peut porter un à trois des restes suivants: nitro, cyano, alkyle en C₁-C₄, halogénoalkyle en C₁-C₄, alcoxy en C₁-C₄, halogénoalcoxy en C₁-C₄, alkylthio en C₁-C₄, cycloalkyle en C₃-C₆ ou hétérocyclyle;
R², R³, R⁴ hydrogène ou, indépendamment l'un de l'autre, une des significations des restes R¹;
n 1;
Y cyano ou halogéno;
W phényle, qui peut porter 1 à 3 des groupes suivants: nitro, halogéno, cyano, alkyle en C₁-C₄, halogénoalkyle en C₁-C₄, alcoxy en C₁-C₄, halogénoalcoxy en C₁-C₄, alkylthio en C₁-C₄, cycloalkyle en C₃-C₆, et alcoxy(C₁-C₄)carbonyle.

2. Amides d'acide cycloalkylalcanecarboxylique de formule générale I 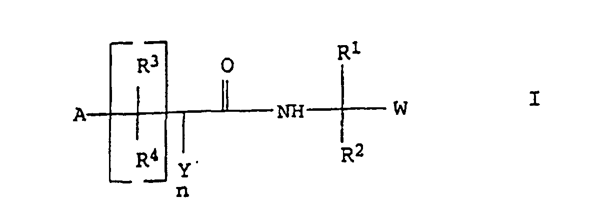 où les substituants ont la signification suivante:
A cycloalkyle en C₃-C₆, qui peut porter un à trois substituants choisis dans le groupe consistant en halogéno et alkyle en C₁-C₃;
R¹ alkyle en C₁-C₆ ou alcényle en C₂-C₆, tandis que ces restes sont partiellement ou totalement halogénés et/ou peuvent porter un ou deux des groupes suivants: alcoxy en C₁-C₄, halogénoalcoxy en C₁-C₄, alkylthio en C₁-C₄, alcoxy(C₁-C₄)carbonyle, cycloalkyle en C₃-C₆ et phényle, tandis que le phényle est partiellement ou totalement halogéné et/ou peut porter un à trois des restes suivants: nitro, cyano, alkyle en C₁-C₄, halogénoalkyle en C₁-C₄, alcoxy en C₁-C₄, halogénoalcoxy en C₁-C₄, alkylthio en C₁-C₄, cycloalkyle en C₃-C₆ ou hétérocyclyle;
R², R³, R⁴ hydrogène ou, indépendamment l'un de l'autre, une des significations du reste R¹;
n 0 ou 1;
Y cyano ou halogéno;
W naphtyle ou hétéroaryle, tandis que ces restes peuvent porter un à trois des groupes suivants: nitro, halogéno, cyano, alkyle en C₁-C₄, halogénoalkyle en C₁-C₄, alcoxy en C₁-C₄, halogénoalcoxy en C₁-C₄, alkylthio en C₁-C₄, cycloalkyle en C₃-C₆, et alcoxy(C₁-C₄)carbonyle;
à l'exception des composés de formule I, où
n 0 et
A est le cyclopentyle.

3. Amides d'acide cycloalkylalcanecarboxylique de formule générale I selon la revendication 1 ou 2, où
A désigne un cyclopropyle, qui peut porter un à trois substituants choisis dans le groupe consistant en halogéno et alkyle en C₁-C₃.

4. Amides d'acide cycloalkylalcanecarboxylique de formule générale I selon l'une des revendications 1 à 3, où A désigne un cyclopropyle qui est substitué par deux atomes de chlore.

5. Amides d'acide cycloalkylalcanecarboxylique de formule générale I selon l'une des revendications 1 ou 2 - 4, où A désigne un cyclopropyle qui est substitué par un groupe alkyle sur l'atome de carbone de la position de jonction du cycle cyclopropane avec le reste de la molécule.

6. Amides d'acide cycloalkylalcanecarboxylique de formule générale I selon l'une des revendications 1 - 5, où
R¹ désigne le méthyle et
R², R³, R⁴ désignent l'hydrogène ou un méthyle.

7. Amides d'acide cycloalkylalcanecarboxylique de formule générale I selon la revendication 2, où
n 1.

8. Procédé pour la préparation d'amides d'acide cycloalkylalcanecarboxylique de formule générale I selon l'une des revendications 1 - 7, 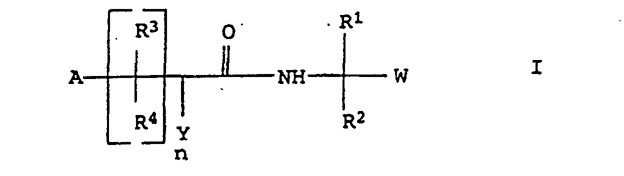 **caractérisé par le fait qu'**on fait réagir un dérivé d'acide carboxylique de formule II, 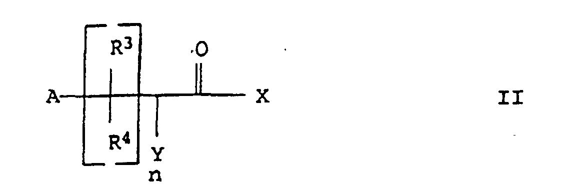 où les restes A, Y, R³ et R⁴, ainsi que n ont la signification indiquée dans la revendication 1 ou 2, et X désigne un reste échangeable par voie nucléophile,
avec une amine de formule III, 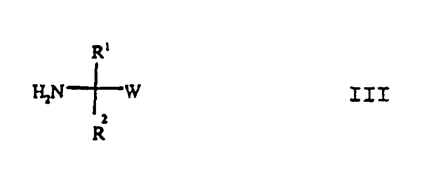 où les restes W, R¹ et R² possèdent la signification indiquée dans la revendication 1.

9. Dérivés d'acide carboxylique de formule IIB, 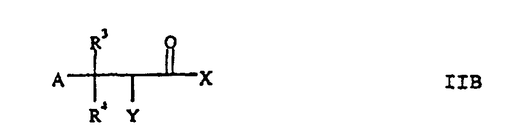 où
X désigne un reste échangeable par voie nucléophile,
A désigne un cycloalkyle en C₃-C₆, qui est substitué par un à trois substituants choisis dans le groupe consistant en halogéno et alkyle en C₁-C₃,
et les restes R³, R⁴ et Y possèdent la signification indiquée dans la revendication 1.

10. Dérivés d'acide carboxylique selon la revendication 9, où A désigne un cyclopropyle qui est substitué par deux atomes de chlore.

11. Dérivés d'acide carboxylique selon des revendications 9 à 10, où A désigne un cyclopropyle qui est substitué par un groupe alkyle sur l'atome de carbone de la position de jonction du cycle cyclopropane avec le reste de la molécule.

12. Produit contenant une quantité efficace pour la lutte contre les champignons nuisibles d'au moins un amide d'acide cycloalkylalcanecarboxylique de formule I selon l'une des revendications 1 à 7 et au moins un support inerte liquide et/ou solide, ainsi qu'éventuellement au moins une substance tensioactive.

13. Procédé pour la lutte contre les champignons nuisibles, **caractérisé par le fait qu'**on traite les champignons nuisibles, leur biotope ou les plantes, surfaces, matériaux ou espaces qui doivent en être libérés avec une quantité efficace d'un amide d'acide cycloalkylalcanecarboxylique de formule I selon l'une des revendications 1 - 7.

14. Utilisation d'un amide d'acide cycloalkylalcanecarboxylique de formule générale I selon l'une des revendications 1 - 7 pour la lutte contre les champignons nuisibles.
